# EUROPEAN PATENT SPECIFICATION

(11) **EP 3 134 515 B1**
(45) Date of publication and mention of the grant of the patent: **27.03.2019**
(21) Application number: 15721095.6
(22) Date of filing: 24.04.2015
(51) Int. Cl.: A61K 39/00, C12N 15/86, G01N 33/566, C12N 5/0789, C12N 5/074

(54) **APPLICATION OF INDUCED PLURIPOTENT STEM CELLS TO GENERATE ADOPTIVE CELL THERAPY PRODUCTS**
ANWENDUNG VON INDUZIERTEN PLURIPOTENTEN STAMMZELLEN ZUR ERZEUGUNG VON ADOPTIVEN ZELLTHERAPIEPRODUKTEN
APPLICATION DE CELLULES SOUCHES PLURIPOTENTES INDUITES POUR GÉNÉRER DES PRODUITS DE THÉRAPIE CELLULAIRE ADOPTIVE

(30) Priority: 24.04.2014 US 201461983722 P
(43) Date of publication of application: 01.03.2017
(73) Proprietor: Board of Regents, The University of Texas System, Austin, TX 78701 (US)
(72) Inventor: COOPER, Laurence, J.N., Houston, TX 77054 (US); TORIKAI, Hiroki, Houston, TX 77054 (US)
(74) Representative: Grünecker Patent- und Rechtsanwälte PartG mbB
(86) International application number: PCT/US2015/027511
(87) International publication number: WO 2015/164740

(56) References cited:
- WO-A2-2014/037807
- HIROKI TORIKAI ET AL: "Toward eliminating HLA class I expression to generate universal cells from allogeneic donors", BLOOD, AMERICAN SOCIETY OF HEMATOLOGY, US, vol. 122, no. 8, 22 August 2013 (2013-08-22), pages 1341-1349, XP002719612, ISSN: 0006-4971, DOI: 10.1182/BLOOD-2013-03-478255 [retrieved on 2013-06-05] cited in the application
- LAURA RIOLOBOS ET AL: "HLA Engineering of Human Pluripotent Stem Cells", MOLECULAR THERAPY, vol. 21, no. 6, 1 June 2013 (2013-06-01), pages 1232-1241, XP055145726, ISSN: 1525-0016, DOI: 10.1038/mt.2013.59 cited in the application
- QIANG FENG ET AL: "Scalable Generation of Universal Platelets from Human Induced Pluripotent Stem Cells", STEM CELL REPORTS, vol. 3, no. 5, 1 November 2014 (2014-11-01), pages 817-831, XP055203082, ISSN: 2213-6711, DOI: 10.1016/j.stemcr.2014.09.010

## Description

### BACKGROUND OF THE INVENTION

### 1. Field of the Invention

The present invention relates generally to the fields of medicine, cell biology, and molecular biology. In certain aspects, the field of the invention concerns immunotherapy. More particularly, it concerns adoptive cell therapy products for immunotherapy and regenerative medicine and for screening of potential toxicity of immune receptors.

### 2. Description of Related Art

Cell therapy using autologous or human leukocyte antigen (HLA)-matched allogeneic donor cells is a promising therapy for many types of diseases, including cancer, and for regenerative medicine. However, autologous cells are sometimes functionally flawed due to the disease itself or repeated administration of toxic drugs, especially in patients with cancer. In the case of allogeneic cells, patients need to find suitable HLA-matched donors to avoid allogeneic immune responses. For example, allogeneic hematopoietic stem cells (HSCs) are infused to restore or replace absent or dysfunctional HSCs in the recipient and serve as a source for generating specific hematopoietic cells. However, the diversity of the HLA system poses a hurdle to finding an HLA-compatible donor, which is exacerbated by the impact of racial genetic polymorphism. In order to provide suitable HLA-matched products for patient, a robust number of donors are needed. Even despite pre-banking umbilical cord blood (UCB) units and access to registered adult donors through the National Marrow Donor Program (NMDP), finding a suitable HLA-matched product remains challenging for many recipients, especially those from racial and ethnic minorities who are under-represented in the donor pool. In fact, the nine million donors registered in the NMDP are not sufficient to cover the U.S. population. Furthermore, it takes significant time and monetary expense to prepare the cell therapy product. As such, a cell product that avoids allogeneic immune cell-mediated rejection and that can be infused into a patient without the need to expand a cell culture from the patient is needed.

Torikai et al., Blood, 2013, 122, 1341-1349, discloses the elimination of HLA class I expression to make allogeneic cells available for multiple recipients.

Riolobos et al., Mol. Ther., 2013, 21, 1232-1241, discloses the generation of HLA homozygous stem cells and Beta-2-Microglobulin knock out stem cells for allogeneic cell transfer.

### SUMMARY OF THE INVENTION

The invention is as defined in the claims.

The present disclosure provides for the application of induced pluripotent stem cells to generate adoptive cell therapy products for immunotherapy regenerative medicine and for screening of potential toxicity of immune receptors.

In one embodiment, a method is provided for producing HLA-A^{neg}, HLA-homozygous induced pluripotent stem cells (iPSCs) comprising (a) obtaining a population of cells from an HLA-homozygous donor; (b) engineering the cells so that they do not express HLA-A, thereby producing HLA-A^{neg} cells; and (c) reprogramming the HLA-A^{neg} cells to generate iPSCs, thereby producing HLA-A^{neg} iPSCs.

In one aspect, the population of cells may be a population of umbilical cord blood cells. In one aspect, the donor may be HLA-homozygous at HLA-B, HLA-C, and HLA-DRB1.

In some aspects, engineering the cells so that they do not express HLA-A may comprise introducing into the cells an artificial nuclease that specifically targets the HLA-A locus. In various aspects, the artificial nuclease may be a zinc finger nuclease, TALEN, or CRISPR/Cas9. In various aspects, introducing into the cells an artificial nuclease may comprise introducing mRNA encoding the artificial nuclease into the cells.

In some aspects, reprogramming cells (*e.g*., HLA-A^{neg} cells) may comprise introducing reprogramming factors selected from the group consisting of Sox2, Oct3/4, Nanog, Lin-28, Klf4, myc *(e.g.,* C-myc, L-myc, or a myc mutant that is deficient in transformation) and/or SV40LT into the cells. In some aspects, reprogramming cells, such as HLA-A^{neg} cells, comprise introducing reprogramming factors Oct3/4, KLF4, Sox2, and c-myc protein into the cells. In further aspects, reprogramming may comprise introducing Oct3/4, KLF4, Sox2, and c-myc encoding mRNA into the cells. In still further aspects, reprogramming may comprise introducing one or more expression cassettes encoding Oct3/4, KLF4, Sox2, and c-myc into the cells. The one or more expression cassettes may be comprised in one or more episomal vectors. The one or more expression cassettes may be comprised in one or more viral vectors (*e.g.,* retroviral vector, lentiviral vector, or Sendai viral vector).

In certain aspects, the method may further comprise introducing into the cells of step (b) a suicide gene, such as, for example, inducible caspase 9 (iCasp9). In some aspects, introducing may comprise gene transfer. In some aspects, introducing may comprise a transposon/transposase system, such as the Sleeping Beauty transposon/transposase system.

In certain aspects, the method may further comprise identifying HLA-A^{neg}, HLA-homozygous iPSCs that have a genetically safe harbor profile. As used herein, a "safe harbor" profile refers to the insertion of foreign genetic material into the genome at sites where transgene expression is sustained (*i.e.*, not silenced) and does not disrupt expression of endogenous genes. For example, a "genetically safe harbor profile" may refer to a transgenic event that is positioned outside of the coding and expression control regions of endogenous genes. In some aspects, identifying may comprise performing whole genome sequencing or integration site analysis.

In certain aspects, the method may further comprise differentiating the HLA-A^{neg}, HLA-homozygous iPSCs. In some aspects, differentiating may comprise the use of artificial antigen presenting cells (aAPC). In some aspects, the aAPC may be genetically-modified K562 cells.

In some aspects, the iPSCs may be differentiated into immune cells, such as, for example, T cells, NK cells, iNKT cells. In some aspects, the immune cells may further comprise a tumor-specific or virus-specific TCRαβ. In some aspects, the immune cells may further comprise a tumor-specific chimeric antigen receptor. In some aspects, the immune cells may be further genetically edited to eliminate immune suppressive molecules (*e.g*., PD-1 or CTLA-4). In some aspects, the iPSCs may be differentiated into hematopoietic stem cells. In some aspects, the iPSCs may be differentiated into cardiomyocytes, lung epithelial cells, beta islet cells, renal cells, or neuronal cells.

In a further embodiment, there is provided an isolated mammalian cell (*e.g.,* a human cell), the cell comprising at least one set of homozygous HLA alleles (*e.g.,* a homozygous HLA-B, HLA-C and/or HLADRB1 alleles) and a HLA-A^{neg} phenotype. In some aspects, a cell having a HLA-A^{neg} phenotype comprises a deletion of all or part of at least one of the HLA-A genes or comprises a mutation in a HLA-A that renders one or both of the genes or gene products non-function. For example, in some aspects, one or both of the HLA-A genes includes a deletion generated by a zinc finger nuclease, TALEN, or CRISPR/Cas9 system that renders the gene or gene product non-functional. In further aspects, a cell of the embodiments that comprises at least one set of homozygous HLA alleles, comprises at least two or three sets of homozygous HLA alleles. For example, the cell can comprise homozygous HLA-B and HLA-C alleles, homozygous HLA-B and HLA-DRB1 alleles, homozygous HLA-C and HLA-DRB1 alleles or homozygous HLA-B, HLA-C and HLA-DRB1 alleles.

In some aspects, an isolated mammalian cell comprising at least one set of homozygous HLA alleles and a HLA-A^{neg} phenotype of the embodiments is a embryonic stem cell, an iPS cell, an umbilical cord blood cell, an epidermal cell, a pancreatic cell, a liver cell, a hematopoietic cell, a mesenchymal cell, a neural cell or an immune cell, such as an NK cell or a T-cell (*e.g.,* a CD4 or CD8 positive T cell). In still further aspects, a population of cells of the embodiments is provided. For example, in some aspects, a population of between about 1 x 10³ and 1 x 10⁴, 1 x 10⁶, 1 x 10⁶, or 1 x 10⁷ cells of the embodiments is provided.

In some aspects, an isolated mammalian cell comprising at least one set of homozygous HLA alleles and a HLA-A^{neg} phenotype of the embodiments further comprises a transgene. For example, the cell may comprise a transgene encoding a reporter, a drug selection marker, a chimeric antigen receptor (CAR) and/or a suicide gene (*e.g*., thymidine kinase or inducible caspase 9 (iCasp9). In still further aspects, a mammalian cell of the embodiments further comprises reduced expression or activity of one or more endogenous gene. For example, in some aspects, the cell lacks expression, or has reduced expression, of one or more immune suppressive molecule, such as PD-1 or CTLA-4. In still further aspects, the cell lacks expression, or has reduced expression, of one or more T cell receptor component. For example, in some aspects, the cell lacks expression or have reduced expression of TCRα, TCRβ or TCRα and TCRβ.

In still a further embodiment, an in vitro set of cell lines is provided comprising at least a first and second induced pluripotent stem cell line, wherein said first and second lines comprise homozygous HLA-B and HLA-C alleles, wherein the homozygous HLA-B and/or HLA-C alleles of the first cell line are different from the homozygous HLA-B and/or HLA-C alleles of the second cell line, wherein the first and second line are both HLA-A^{neg}. In certain aspects, the set of cell lines may further comprise at least five to ten different induced pluripotent stem cell lines, wherein each line comprises a unique combination of homozygous HLA-B and HLA-C alleles, wherein each line is HLA-A^{neg}. In certain aspects, the set of cell lines may further comprise at least twenty different induced pluripotent stem cell lines, wherein each line comprises a unique combination of homozygous HLA-B and HLA-C alleles, wherein each line is HLA-A^{neg}. In certain aspects, the set of cell lines may further comprise at least twenty-seven different induced pluripotent stem cell lines, wherein each line comprises a unique combination of homozygous HLA-B and HLA-C alleles, wherein each line is HLA-A^{neg}. In one aspects, the set of cell lines may comprise twenty-seven different induced pluripotent stem cell lines, wherein each lines comprises a unique combination of homozygous HLA-B and HLA-C alleles according to Table 1, wherein each line is HLA-A^{neg}.

In some aspects, each line may further comprise a suicide gene, such as inducible caspase 9 (iCasp9). In some aspects, the cell lines may be produced according to a method of the present embodiments.

In one embodiment, a method is provided for screening immune receptor specificity comprising (a) obtaining an *in vitro* set of iPSC lines according to the present embodiments; (b) optionally differentiating the iPSCs into lineage-specific cells; (c) exposing the iPSCs or lineage-specific cells to T cells expressing an immune receptor of interest; and (d) detecting an interaction between the iPSCs or lineage-specific cells and the T cells expressing an immune receptor of interest, thereby screening for immune receptor specificity.

In some aspects, the T cells may be a T-acute lymphoblastic leukemia cell line that expresses GFP only after recognizing the target antigen for the immune receptor of interest. In this aspect, step (d) may comprise detecting the expression of GFP in the T-acute lymphoblastic leukemia cell line, thereby identifying iPSCs or lineage-specific cells that express the target antigen.

In some aspects, the method may further comprise introducing into the cells of step (a) a cell death reporter construct. In some aspects, the cell death reporter construct may allow for detection of caspase-3 activation. In these aspects, step (d) may comprise detecting activation of the cell death reporter construct, thereby screening for immune receptor specificity.

In some aspects, the method may further comprise introducing into the cells of step (a) a lineage-specific transcription factor promoter-driven reporter gene. In this aspect, step (d) may comprise detecting a loss of expression of the reporter gene, thereby screening for immune receptor specificity.

In some aspects, the immune receptor of interest may be a T-cell receptor (TCR) or chimeric antigen receptor (CAR). In some aspects, the TCR may be cloned or manipulated.

In one embodiment, a method is provided for treating a disease in a patient in need thereof comprising (a) selecting a cell line from an *in vitro* set of cell lines according to any one of the present embodiments that is HLA-matched to the patient; (b) differentiating the selected cell line to lineage-specific cells; and (c) administering a therapeutically effective amount of the differentiated cells to the patient.

In certain aspects, the method may be a method of providing immunotherapy. In some aspects, the lineage-specific cells may be hematopoietic stem cells or immune effector cells. In some aspects, the disease may be cancer, an autoimmune disease, or an infectious disease. In some aspects, the immune effector cells may be T cells, NK cells, and iNKT cells.

In certain aspects, the method may further comprise introducing into the cells of step (a) a chimeric antigen receptor (CAR) or T-cell receptor. In some aspects, the disease may be cancer and the CAR may be targeted to a cancer cell antigen, such as, for example, CD19, CD20, carcinoembryonic antigen, alphafetoprotein, CA-125, 5T4, MUC-1, epithelial tumor antigen, melanoma-associated antigen, mutated p53, mutated ras, HER2/Neu, ERBB2, folate binding protein, GD2, CD123, CD23, CD30, CD56, c-Met, meothelin, GD3, HERV-K, IL-11Ralpha, kappa chain, lambda chain, CSPG4, ERBB2, EGFRvIII, or VEGFR2.

In some aspects, the disease may be an autoimmune disease and the CAR may be targeted to the autoimmune cells. Examples of autoimmune diseases include, but are not limited to, Coeliac disease, diabetes mellitus type 1 (IDDM), systemic lupus erythematosus (SLE), Sjögren's syndrome, multiple sclerosis (MS), Hashimoto's thyroiditis, Graves' disease, idiopathic thrombocytopenic purpura, rheumatoid arthritis (RA), acute idiopathic thrombocytopenic purpura, chronic idiopathic thrombocytopenic purpura, dermatomyositis, Sydenham's chorea, myasthenia gravis, systemic lupus erythematosus, lupus nephritis, rheumatic fever, polyglandular syndromes, bullous pemphigoid, diabetes mellitus, Henoch-Schonlein purpura, post-streptococcalnephritis, erythema nodosum, Takayasu's arteritis, Addison's disease, rheumatoid arthritis, multiple sclerosis, sarcoidosis, ulcerative colitis, erythema multiforme, IgA nephropathy, polyarteritis nodosa, ankylosing spondylitis, Goodpasture's syndrome, thromboangitisubiterans, Sjogren's syndrome, primary biliary cirrhosis, Hashimoto's thyroiditis, thyrotoxicosis, scleroderma, chronic active hepatitis, polymyositis/dermatomyositis, polychondritis, pamphigus vulgaris, Wegener's granulomatosis, membranous nephropathy, amyotrophic lateral sclerosis, tabes dorsalis, giant cell arteritis/polymyalgia, perniciousanemia, rapidly progressive glomerulonephritis, psoriasis, and fibrosing alveolitis.

In some aspects, the disease may be an infectious disease caused by a pathogen (*e.g.* a fungal, viral, or bacterial pathogen) and the CAR may be targeted to a pathogen antigen.

In certain aspects, the method may be a method of providing regenerative medicine. In this aspect, the lineage-specific cells may be cardiomyocytes, neurons, beta islet cells, kidney cells, lung cells, epidermal cells, pancreatic cells, liver cells, hematopoietic cells or mesenchymal cells.

In some aspects, a method of the embodiments further comprises obtaining, producing or using antigen presenting cells (APCs). In some aspects, the APCs can be dendritic cells. In certain aspects, the APCs artificial antigen presenting cells (aAPCs), which have been engineered for function as APCs. For example, in some aspects, the aAPCs are inactivated, such as by irradiation. Methods for producing such APCs are known in the art and further detailed herein. Thus, in some aspects, cells of the embodiments (*e.g*., immune cells having a HLA-A^{neg} phenotype) are co-cultured with APCs *ex vivo* for a limited period of time in order to expand the T cell population. The step of co-culturing cells with APCs can be done in a medium that comprises, for example, interleukin-21 (IL-21) and/or interleukin-2 (IL-2). In some aspects, the co-culturing is performed at a ratio of immune cells (*e.g.,* T cells) to APCs of about 10:1 to about 1:10; about 3:1 to about 1:5; or about 1:1 to about 1:3. For example, the co-culture of immune cells and APCs can be at a ratio of about 1:1, about 1:2 or about 1:3.

In some embodiments, methods are provided for treating an individual with a medical condition comprising the step of providing an effective amount of cells from a population of cells described herein, including more than once in some aspects, such as at least 1, 2, 3, 4, 5, 6, 7, 8, 9, 10, 11, 12, 13, 14, or more days apart. In specific embodiments, the medical condition is cancer or an infectious disease.

As used herein a "HLA-A^{neg}" phenotype refers to cell, which does express functional HLA-A on its surface. For example, a cell having a HLA-A^{neg} phenotype may comprises a deletion of all or part of one or both of the HLA-A genes. For example, the HLA-A gene(s) may comprise a deletion introduced by a zinc finger nuclease, TALEN, or CRISPR/Cas9 system. Likewise, in some aspects, a cell having a HLA-A^{neg} phenotype may comprises a mutation in one or both HLA-A genes that renders the gene or gene product non-functional.

As used herein, "essentially free," in terms of a specified component, is used herein to mean that none of the specified component has been purposefully formulated into a composition and/or is present only as a contaminant or in trace amounts. The total amount of the specified component resulting from any unintended contamination of a composition is therefore well below 0.05%, preferably below 0.01%. Most preferred is a composition in which no amount of the specified component can be detected with standard analytical methods.

As used herein in the specification and claims, "a" or "an" may mean one or more. As used herein in the specification and claims, when used in conjunction with the word "comprising", the words "a" or "an" may mean one or more than one. As used herein, in the specification and claim, "another" or "a further" may mean at least a second or more.

As used herein in the specification and claims, the term "about" is used to indicate that a value includes the inherent variation of error for the device, the method being employed to determine the value, or the variation that exists among the study subjects.

Other objects, features and advantages of the present invention will become apparent from the following detailed description. It should be understood, however, that the detailed description and the specific examples, while indicating certain embodiments of the invention, are given by way of illustration only.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.
**FIGS. 1A-D****.** The probability of finding HLA-matched donors in NMDP. FIG. 1A. 8/8 Match. FIG. 1B. 6/6 without HLA-A. FIG. 1C. 6/6 without HLA-B. FIG. 1D. 6/6 without HLA-C. The lower portion of each bar shows the probability of finding 8/8 allele matched donors. The top portion of each bar shows the probability of finding 6/6 allele matched donors if the corresponding HLA locus is eliminated from current HLA matching requirements. X-axis shows the ethnic group.
**FIG. 2****.** Screening toxicities of immune receptors using iPSCs. iPSC will be differentiated into lineage-specific cells directly (left panel) or after modification with a death reporter gene (middle panel) or a lineage-specific reporter gene (right panel). Potential recognition of differentiated cells will be monitored using reporters to detect immune receptor-mediated recognition (left panel) or by co-culturing with immune receptor-expressing T cells.
**FIG. 3****.** Diagram illustrating a method of treating disease by differentiating HLA-A^{neg} HLA-B/C/DRB1 homozygous iPSCs.
**FIGS. 4A-B.** SeV vector mediated reporter gene expression. FIG. 4A. Activated T-cells were infected with SeV vector encoding GFP at designated MOI and cell number. Flow cytometry data on Day 1 are shown. X-axis: GFP expression, Y-axis: CD3. MFI: mean fluorescence intensity. Numbers in right upper quadrants represent percentage of CD3+GFP+ cells. FIG. 4B. GFP expression from Day1 to Day7. MFI were measured each day and plotted on the graph. Solid square: no SeV, Open circle: MOI=1, 1 × 10⁶, Closed circle: MOI=3, 1 × 10⁶, Open diamond: MOI=1,5 × 10⁵, Closed diamond: MOI=3, 5 × 10⁵.
**FIG. 5****.** Reprogramming T cells into iPSCs. Phase contrast view of iPSC. On Day 16, live staining of TRA-1-60 was performed with Alexa Fluor 647 anti-TRA-1-60 antibody.
**FIG. 6****.** Immunofluorescence staining of iPS colonies. After fixation, iPS colonies were stained with the designated antibodies and Alexa 488 conjugated secondary antibodies. DAPI staining was performed immediately before imaging with a fluorescence microscope.
**FIG. 7****.** Nucleofection of iPSCs. DNA plasmid or mRNA encoding eGFP reporter were introduced into iPSC by Nucleofection. Briefly, one million completely dissociated iPSCs were resuspended in 20 µL of P3 buffer, mixed with mRNA or DNA, transfered to strip, and nucleofected using a 4D Nucleofector (Program: CA-137). GFP expression on Day 1 in iPSCs was evaluated by flow cytometry. Data shown are gated for the TRA-1-60 positive population.
**FIG. 8****.** Disruption of the TRAC and TRBC gene by zinc finger nuclease. The arrows in the gel picture represent digested PCR products mediated by Cel-1 enzyme, indicating that TRAC and TRBC targeted ZFNs introduce desired mutation at ZFN target sites.
**FIG. 9****.** Analysis of TRAC gene disruption by sequencing. WT = SEQ ID NO: 1; #13-1 = SEQ ID NO: 2; #13-2 = SEQ ID NO: 3.
**FIG. 10****.** Disruption of the TCR gene by ssODN+ZFN. To introduce stop codon within TRAC target site, single strand oligonucleotides were introduced along into cells with TRAC targeted ZFN. After culture for 10-14 days, the intended mutation at ZFN target site was detected by digital droplet PCR.
**FIG. 11****.** Constructs for CAR introduction by Sleeping Beauty and Lentivirus. The figure shows a schematic representation of the plasmids for SB system and Lentivirus to introduce iCasp9 and CD19 targeted CAR.
**FIG. 12****.** Analysis of CAR introduction by Sleeping Beauty and Lentivirus.
**FIG. 13****.** Protocol for and analysis of re-differentiation of T cells from iPS cells. For *in vitro* differentiation of hematopoietic stem cells from iPSCs, embryoid bodies were formed in culture in an AggreWell™ plate, including appropriate cytokines. On day 13 CD34^{pos} and CD43^{pos} hematopoietic stem cells were detected by flow cytometry.

### DESCRIPTION OF ILLUSTRATIVE EMBODIMENTS

### I. Aspects of the Embodiments

The application of induced pluripotent stem cells (iPSCs) to generate adoptive cell therapy products applicable a large patient population are described herein. Usage of such iPSCs for screening potential toxicity (*e.g.,* of immune receptors) is also described. Cells detailed herein provide therapeutic reagents for the use in treating a wide range of disease. In particular, the cells include modified HLA gens that render their use permissible inn a much wider patient population than could otherwise be treated. In particular aspects, cells of the embodiments are homozygous at one or more pairs of HLA alleles and comprise a HLA-A^{neg} phenotype. These features allow the cells to be effective immunological matches for a large number of patients thereby providing a cell-based therapies that can be produced at a much lower cost than therapies developed for individual patients.

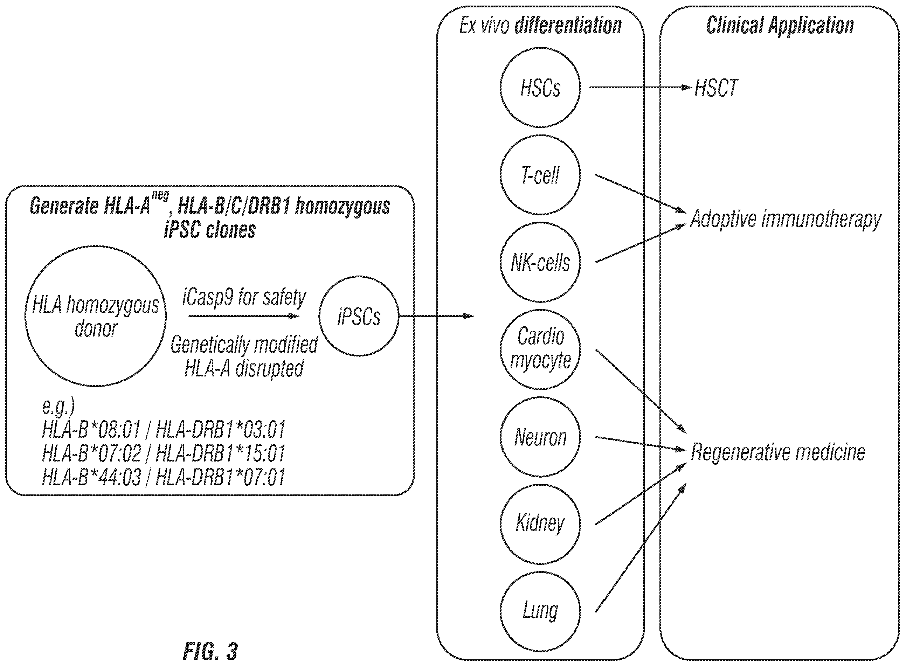

As outlined in FIG. 3, HLA-A^{neg} cells, such as iCasp9⁺HLA-A^{neg} cells, can be generated from HLA homozygous cells by selective disruption of the HLA-A coding gene. For example, HLA homozygous umbilical cord blood cells by genetic modification with i) Sleeping Beauty (SB) transposon/transposase system (or other gene transfer approaches) to introduce iCasp9 genes (or other suicide gene) and ii) zinc finger nucleases (ZFNs) or other artificial nucleases (*e.g*., TALEN, CRISPR/Cas9) to eliminate HLA-A. These cells can be reprogrammed to iPSC by introduction of reprogramming factors (*e.g*., using a Sendai virus encoding Oct3/4, KLF4, Sox2, and c-myc). Alternatively, iPSC clones may be directly genetically modified with SB and ZFNs, in other words, the cells may be reprogrammed first and then genetically modified. After isolating the iPSC clones that have been identified to have a genetically safe harbor profile, the cells can be differentiated into lineage specific cells, which may be administered to patients for immunotherapy and/or regenerative medicine. One approach to differentiating iPSC involves the use of artificial antigen presenting cells (aAPC), such as those derived from genetically modified K-562 cells.

### A. Generating HLA-A^{neg} HLA-homozygous iPSCs and Methods of Use Thereof

Embodiments of the present invention provide an allogeneic iPSC bank for allogeneic cell therapies for immunotherapy and regenerative medicine. Generating a characterized product that can be pre-prepared and infused on demand has enormous appeal as it enables centralized manufacturing and delivery of cell-based therapies when needed rather than when available. Furthermore, the genetic manipulation of iPSC and the characterization of clones will enable down-stream clinical-grade products to be generated. This technology will hasten the cell therapy process by allowing clinicians to infuse allogeneic therapeutic cells on demand, reduce donor-to-donor heterogeneity, and also reduce the cost to prepare cell therapy products.

Methods to improve access to the existing donor pool are anticipated to have clinical impact. In fact, the National Marrow Donor Program (NMDP) calculates that approximately 10,000 patients in the U.S. could benefit from unrelated allogeneic hematopoietic stem cell transplant (HSCT). The clinical application of donors already registered would be markedly increased if allogeneic HSCs were genetically edited to eliminate expression of the HLA-A locus. Modeling from the NMDP shows that the chance of an African American recipient finding a HLA-matched donor increases from 18% to 73% when the need for a match at HLA-A is eliminated and leaving only HLA-B, -C, and -DR. Engineered zinc finger nucleases (ZFNs) (or any other artificial nuclease, *e.g*., TALEN, CRISPR/Cas9) can eliminate HLA-A expression in genetically edited T cells and cell lines. The elimination of HLA-A expression in HSCs using ZFNs provides an appealing approach to increase the chance for finding HLA-matched donors. This is anticipated to broaden the clinical application of allogeneic HSCT, especially for patients belonging to ethnic minority groups.

The generation of banks of HLA-A^{neg} HLA-homozygous iPSCs will solve these problems as they can be infused into multiple recipients. This can be accomplished by knocking out HLA-A (*e.g*., using designer zinc finger nuclease, TALEN, CRISPR/Cas9) from approximately 27 unique donors (homozygous at HLA-B, -C, and -DRB1, see Table 1), such as iPSCs derived from umbilical cord blood. These iPSC banks can be engineered to insert as well as remove genes to improve immunotherapy and regenerative medicine. These HLA-A^{neg} iPSCs and cells differentiated therefrom (*e.g*., immune cells, cardiac cells, kidney cells) will be a very valuable resource that can be used to generate immune cells as well as other therapeutic cells, such as, for example, but not limited to, cardiac cells, pancreas, kidney, NK cells, iNKT cells, and T cells, including sub-types. This will generate therapy for illnesses (*e.g*., cancer, autoimmune disease, infection) as well as regenerative medicine.

**Table 1. Unique HLA-B, HLA-C, and HLA-DRB1 homozygotes needed to cover the U.S. population.**

| **Rank** | **HLA-B Allele** | **HLA-C Allele** | **HLA-DRB1 Allele** | **EUR_freq** | **Population coverage** | **Cumulative population coverage** |
|---|---|---|---|---|---|---|
| 1 | 0801g | 0701g | 301 | 0.10285 | 0.2057 | 0.2057 |
| 2 | 702g | 702 | 1501 | 0.08171 | 0.16342 | 0.36912 |
| 3 | 4402g | 0501g | 401 | 0.03118 | 0.06236 | 0.43148 |
| 4 | 4403 | 1601 | 701 | 0.02785 | 0.0557 | 0.48718 |
| 5 | 3501g | 0401g | 101 | 0.02745 | 0.0549 | 0.54208 |
| 6 | 5701 | 602 | 701 | 0.0248 | 0.0496 | 0.59168 |
| 7 | 1302 | 602 | 701 | 0.02077 | 0.04154 | 0.63322 |
| 8 | 1501g | 304 | 401 | 0.01745 | 0.0349 | 0.66812 |
| 9 | 4001g | 304 | 401 | 0.01423 | 0.02846 | 0.69658 |
| 10 | 1402 | 802 | 102 | 0.01363 | 0.02726 | 0.72384 |
| 11 | 4001g | 304 | 1302 | 0.01337 | 0.02674 | 0.75058 |
| 12 | 4403 | 0401g | 701 | 0.01144 | 0.02288 | 0.77346 |
| 13 | 1501g | 0303g | 1301 | 0.01039 | 0.02078 | 0.79424 |
| 14 | 1801g | 0501g | 301 | 0.00996 | 0.01992 | 0.81416 |
| 15 | 0702g | 702 | 101 | 0.00984 | 0.01968 | 0.83384 |
| 16 | 4402g | 0501g | 1301 | 0.00889 | 0.01778 | 0.85162 |
| 17 | 4402g | 0501g | 1501 | 0.00785 | 0.0157 | 0.86432 |
| 18 | 3801 | 1203 | 1301 | 0.00745 | 0.0149 | 0.88222 |
| 19 | 2705g | 102 | 101 | 0.00741 | 0.01482 | 0.89704 |
| 20 | 3502 | 0401g | 1104 | 0.00699 | 0.01398 | 0.91102 |
| 21 | 1401 | 802 | 701 | 0.00691 | 0.01382 | 0.92484 |
| 22 | 1801g | 0701g | 1104 | 0.00662 | 0.01324 | 0.93808 |
| 23 | 5201g | 1202 | 1502 | 0.00657 | 0.01314 | 0.95122 |
| 24 | 0801g | 0701g | 1501 | 0.00639 | 0.01278 | 0.964 |
| 25 | 1801g | 1203 | 1501 | 0.00629 | 0.01258 | 0.97658 |
| 26 | 4402g | 0501g | 101 | 0.0061 | 0.0122 | 0.98878 |
| 27 | 3801 | 1203 | 402 | 0.00587 | 0.01174 | 1.00052 |

However, the endogenous αβ T-cell receptor (TCR) on infused allogeneic T cells may recognize major and minor histocompatibility antigens in the recipient, leading to graft-versus-host-disease (GVHD). To overcome this, one can eliminate endogenous TCR αβ and γδ expression, which causes unwanted allogeneic immune reaction. Such steps can occur by any suitable manner, including by introducing zinc finger nucleases (ZFN), for example, targeting TCR α constant region or β constant region.

### B. Using iPSC to Prevent Off-target Toxicity and Recognition by Recipient

Current screening methods to identify expression of target antigens are limited. Embodiments of the present invention provide for the screening of iPSC/iPSC-derived cells, which will solve the current issues for predicting potential toxicity of immune receptors. As such, the banks of HLA-A^{neg} HLA-homozygous iPSCs can be used as a source of cells to screen for toxicity arising from immune cells engineered with potential for immunotherapy. Screening for unintended adverse events (on-target as well as off-target toxicity) using iPSC will improve adoptive T-cell therapy (as well as therapy infusing other immune cells) by decreasing the possibility of an un-predictable off-target toxicity by an endogenous or introduced immune receptor. For example, prior to their human application, cloned/manipulated T-cell receptors (TCRs) and chimeric antigen receptors (CARs) need to be screened for potential toxicity. This can be accomplished *in vitro* using panels of engineered iPSC and their differentiated progeny.

### II. Cells of the Embodiments

In certain embodiments, somatic cells are reprogrammed by the introduction of reprogramming factors to generate induced pluripotent stem cells. The progeny of these cells could be identical to embryonic stem cells in various aspects as described below and as are well known in the art. Understanding of embryonic stem cell characteristics could help select induced pluripotent stem cells produced by these methods. Reprogramming factors known from stem cell reprogramming studies may be used for these methods.

The term "cell" is used herein in its broadest sense in the art and refers to a living body that is a structural unit of tissue of a multicellular organism, is surrounded by a membrane structure that isolates it from the outside, has the capability of self replicating, and has genetic information and a mechanism for expressing it. Cells used herein may be naturally-occurring cells or artificially modified cells (*e.g.,* fusion cells, genetically modified cells, *etc.*)*.*

The term "differentiated cell" as used herein can refer to a cell that has developed from an unspecialized phenotype to a specialized phenotype. For example, embryonic cells can differentiate into an epithelial cell of the intestinal lining. Differentiated cells can be isolated from a fetus or a live born animal, for example.

The term "undifferentiated cell" as used herein can refer to a precursor cell that has an unspecialized phenotype and is capable of differentiating. An example of an undifferentiated cell is a stem cell.

As used herein, the term "stem cell" refers to a cell capable of self replication and pluripotency. "Pluripotent" implies that a cell is capable, through its progeny, of giving rise to all the cell types that comprise the adult animal, including the germ cells and all three germ layers: endoderm (interior stomach lining, gastrointestinal tract, the lungs), mesoderm (muscle, bone, blood, urogenital), or ectoderm (epidermal tissues and nervous system). Stem cells herein may be, but are not limited to, embryonic stem (ES) cells, tissue stem cells (also called tissue-specific stem cells, or somatic stem cells), or induced pluripotent stem cells. Any artificially produced cell that has the above-described abilities (*e.g*., fusion cells, reprogrammed cells, or the like used herein) may be a stem cell.

The term "embryonic stem (ES) cell" as used herein can refer to pluripotent cells isolated from an embryo that are maintained in *in vitro* cell culture. Such cells are rapidly dividing cultured cells isolated from cultured embryos that retain in culture the ability to give rise, *in vivo,* to all the cell types that comprise the adult animal, including the germ cells. Embryonic stem cells may have a rounded cell morphology and may grow in rounded cell clumps on feeder layers. Embryonic stem cells are well known to a person of ordinary skill in the art.

Tissue stem cells are separated into categories based on the sites from which the cells are derived, such as the dermal system (*e.g*., epidermal stem cells, hair follicle stem cells, *etc.*)*,* the digestive system (*e.g.,* pancreatic (common) stem cells, liver stem cells, *etc.*)*,* the bone marrow system (*e.g.,* hematopoietic stem cells, mesenchymal stem cells, *etc.*)*,* the nervous system (*e.g.,* neural stem cells, retinal stem cells, *etc.*)*,* and the like.

"Induced pluripotent stem cells," commonly abbreviated as iPS cells or iPSCs, refer to a type of pluripotent stem cell artificially prepared from a non-pluripotent cell, typically an adult somatic cell, or terminally differentiated cell, such as a fibroblast, a hematopoietic cell, a myocyte, a neuron, an epidermal cell, or the like, by expressing reprogramming factors.

"Self-renewal" refers to the ability to go through numerous cycles of cell division while maintaining the undifferentiated state.

As used herein, the term "somatic cell" refers to any cell other than germ cells, such as an egg, a sperm, or the like, which does not directly transfer its DNA to the next generation. Typically, somatic cells have limited or no pluripotency. Somatic cells used herein may be naturally-occurring or genetically modified.

The term "cultured" as used herein in reference to cells can refer to one or more cells that are undergoing cell division or not undergoing cell division in an *in vitro* environment. An *in vitro* environment can be any medium known in the art that is suitable for maintaining cells *in vitro,* such as suitable liquid media or agar, for example.

The medium according to the present embodiments can be a serum-containing or serum-free medium. The serum-free medium refers to media with no unprocessed or unpurified serum, and accordingly can include media with purified blood-derived components or animal tissue-derived components (such as growth factors). From the aspect of preventing contamination with heterogeneous animal-derived components, serum can be derived from the same animal as that of the stem cell(s).

The medium according to the present embodiments may contain or may not contain any alternatives to serum. The alternatives to serum can include materials that appropriately contain albumin (such as lipid-rich albumin, albumin substitutes such as recombinant albumin, plant starch, dextrans and protein hydrolysates), transferrin (or other iron transporters), fatty acids, insulin, collagen precursors, trace elements, 2-mercaptoethanol, 3'-thiolgiycerol, or equivalents thereto.

### A. Stem Cells

Stem cells are cells found in most, if not all, multi-cellular organisms. They are characterized by the ability to renew themselves through mitotic cell division and differentiating into a diverse range of specialized cell types. The two broad types of mammalian stem cells are: embryonic stem cells that are found in blastocysts and adult stem cells that are found in adult tissues. In a developing embryo, stem cells can differentiate into all of the specialized embryonic tissues. In adult organisms, stem cells and progenitor cells act as a repair system for the body, replenishing specialized cells, but also maintain the normal turnover of regenerative organs, such as blood, skin or intestinal tissues.

As stem cells can be grown and transformed into specialized cells with characteristics consistent with cells of various tissues, such as muscles or nerves, through cell culture, their use in medical therapies has been proposed. The reprogramming of adult cells into induced pluripotent stem cells has enormous potential.

Nearly all research to date has taken place using mouse embryonic stem cells (mES) or human embryonic stem cells (hES). Both have the essential stem cell characteristics, yet they require very different environments to maintain an undifferentiated state. Mouse ES cells may be grown on a layer of gelatin and require the presence of Leukemia Inhibitory Factor (LIF). Human ES cells may be grown on a feeder layer of mouse embryonic fibroblasts (MEFs) and often require the presence of basic Fibroblast Growth Factor (bFGF or FGF-2). Without optimal culture conditions or genetic manipulation (Chambers *et al.,* 2003), embryonic stem cells will rapidly differentiate.

A human embryonic stem cell may be also defined by the presence of several transcription factors and cell surface proteins. The transcription factors Oct4, Nanog, and Sox2 form the core regulatory network that ensures the suppression of genes that lead to differentiation and the maintenance of pluripotency (Boyer *et al.,* 2005). The cell surface antigens most commonly used to identify hES cells include the glycolipids SSEA3 and SSEA4 and the keratan sulfate antigens Tra-1-60 and Tra-1-81.

### B. Reprogramming Factors

The generation of iPS cells is dependent on the genes used for induction. The following factors or combination thereof could be used: Oct3/4, KLF4, Sox2, and/or c-myc. Nucleic acids encoding these reprogramming factors may be comprised in monocistronic or polycistronic expression cassettes. Likewise, nucleic acids encoding said monocistronic or polycistronic expression cassettes may be comprised in one reprogramming vector or multiple reprogramming vectors.

Oct3/4 plays a crucial role in maintaining pluripotency. The absence of Oct3/4 in Oct3/4⁺ cells, such as blastomeres and embryonic stem cells, leads to spontaneous trophoblast differentiation, and presence of Oct3/4 gives rise to the pluripotency and differentiation potential of embryonic stem cells.

KLF4 of the Klf family of genes was initially identified as a factor for the generation of mouse iPS cells and was later demonstrated to be a factor for generation of human iPS cells. Klf2 and Klf4 were found to be factors capable of generating iPS cells, and related genes Klf1 and Klf5 did as well, although with reduced efficiency.

The Sox family of genes is associated with maintaining pluripotency similar to Oct3/4, although it is associated with multipotent and unipotent stem cells in contrast with Oct3/4, which is exclusively expressed in pluripotent stem cells. While Sox2 was the initial gene used for induction by Yamanaka *et al.* (2007), Jaenisch *et al.* (1988) and Yu *et al.* (2007), other genes in the Sox family have been found to work as well in the induction process. Sox1 yields iPS cells with a similar efficiency as Sox2, and genes Sox3, Sox15, and Sox18 also generate iPS cells, although with decreased efficiency.

The Myc family of genes are proto-oncogenes implicated in cancer. Yamanaka *et al.* (2007) and Jaenisch *et al.* (1988) demonstrated that c-myc is a factor implicated in the generation of mouse iPS cells and Yamanaka *et al.* (2007) demonstrated it was a factor implicated in the generation of human iPS cells. N-myc and L-myc have been identified to induce pluripotentcy with similar efficiency to c-myc.

In certain embodiments, in addition to introducing the cells with one or more reprogramming factors, the cells are treated with a reprogramming medium comprising: an HDAC inhibitor, a Wnt pathway inhibitor, an Oct4 activator, hydrocortisone, stem cell factor, EPO, IL-3, CHIR99021, A-83-01, hLIF, and/or bFGF. The use of such molecules may result in improved reprogramming efficiency and kinetics, thereby facilitating iPS cell identification in the primary reprogramming culture and preserving iPS cell clonality.

### C. Methods for Generating Induced Pluripotent Stem Cells

iPS cells are typically derived by transfection of certain stem cell-associated genes into non-pluripotent cells, such as adult fibroblasts or umbilical cord blood cells. Transfection may be achieved through integrating viral vectors, such as retroviruses, or non-integrating viral vectors, such as Sendai virus. After a critical period, small numbers of transfected cells start to become morphologically and biochemically similar to pluripotent stem cells, and may be isolated through morphological selection, doubling time, reporter gene expression, and/or antibiotic resistance.

After the pioneering generation of induced pluripotent stem cells (iPSCs) using retroviral vectors encoding KLF4, cMYC, OCT4, SOX2, various methods have been developed to introduce these factor into many kinds of cells. Among these, Sendai virus (SeV) vectors have been used to effectively generate iPSC from differentiated T cells. SeV is an enveloped virus with a single-stranded, negative-sense, nonsegmented RNA genome of ∼ 15 kb. Recombinant Sendai virus vectors replicate only in the cytoplasm of infected cells and do not go through a DNA phase or integrate into the host genome. SeV vector will diminish after reprogramming T cells into iPSC during their maintenance.

The process for the generation of induced pluripotent stem cells is slow and inefficient, with most cells failing. To improve the efficiency of this process, vectors utilizing a construct containing a reprogramming factor and, optionally, a reporter may be used to optimize transfection efficiency by first selecting for the presence of reporters and later selecting against reporter gene expression as transgene silencing is very effective in pluripotent cells.

Forced persistent expression of ectopic reprogramming factors may be linked to an elevated frequency of tumor formation and a solution to this problem is the generation of transgene-free iPS cells. A suite of introduced genes may be necessary to start the reprogramming process, but gradually the cell's own endogenous pluripotency genes become active, and the introduced genes are silenced with the potential for stochastic reactivation. Exogenous factors may be required for a minimum of about 10-16 days for cells to enter a self-sustaining pluripotent state (Brambrink *et al.,* 2008; Stadtfeld *et al.,* 2008). The determination of the minimum length of transgene expression permits the development of non-integrating delivery methods to derive iPS cells.

Induction of pluripotent stem cells from human somatic cells has been achieved using retroviral or lentiviral vectors for ectopic expression of reprogramming genes. Recombinant retroviruses, such as the Moloney murine leukemia virus, have the ability to integrate into the host genome in a stable fashion due to the function of a reverse transcriptase. Lentiviruses are a subclass of retroviruses. They are widely adapted as vectors owing to their ability to integrate into the genome of non-dividing as well as dividing cells. Methods from the reprogramming of iPS cells using viral vectors are well known in the art.

The use of an integrating viral vector may cause mutation of an endogenous gene or may cause activation of an endogenous oncogene since the viral vector mediates the integration of a gene into a random position of the host cell's chromosome. Therefore, iPS cells may be prepared without genome integrating reprogramming factors. Such methods are described in U.S. Patent Nos. 8,546,140 and 8,268,620. For example, episomal vector elements, including EBV or variants thereof, may be used. In further aspects, episomal vector elements in the iPS cells may be removed after reprogramming.

One possible way to avoid introducing exogenous genetic modifications to target cells would be to deliver the reprogramming proteins directly into cells, rather than relying on the transcription from delivered genes. Previous studies have demonstrated that various proteins can be delivered into cells in vitro and in vivo by conjugating them with a short peptide that mediates protein transduction, such as HIV Tat and poly-arginine. A recent study demonstrated that murine fibroblasts can be fully reprogrammed into pluripotent stem cells by direct delivery of recombinant reprogramming proteins (Zhou *et al.,* 2009).

The starting cell and the differentiated cell generally have differing requirements for culture medium and conditions. It is usual to carry out at least an initial stage of culture, after introduction of the differentiation factors, in the presence of medium and under culture conditions known to be suitable for growth of the starting cell. This is followed by a subsequent period of culture in the presence of a differentiation medium and under conditions known to be suitable for the differentiated cell. After a sufficient time for differentiation, the differentiated cells may be further cultured for expansion of the differentiated cells in an expansion medium. Such an expansion medium may comprise one or more signaling inhibitors or comprise a culture medium essentially free of these inhibitors. The differentiation conditions may be essentially free of feeder cells. In further aspects, the differentiation medium may be chemically defined.

### D. Selection of Reprogrammed Cells

In certain aspects of the embodiments, after a reprogramming vector is introduced into somatic cells, these cells will be cultured for expansion. Cells may be selected for the presence of vector elements, such as reporters or selection markers, to concentrate transfected cells. Reprogramming vectors will express reprogramming factors in these cells and replicate and partition along with cell division. These expressed reprogramming factors will reprogram somatic cell genome to establish a self-sustaining pluripotent state, and in the meantime or after removal of positive selection of the presence of vectors, exogenous genetic elements will be lost gradually in embodiments where non-integrating vectors are used. This silencing of transgene expression permit selection of induced pluripotent stem cells by turning off reporter gene expression alone or in combination with selection for other embryonic stem cell characteristics (e.g., morphology, growth properties, stem cell markers, stem cell genes, telomerase activity, pluripotency, teratoma formation, embryoid body formation, blastocyst injection, promoter demethylation, and histone demethylation) because they are expected to be substantially identical to pluripotent embryonic stem cells. An additional negative selection step could be also employed to accelerate or help selection of iPS cells essentially free of exogenous genetic elements by testing the absence of reprogramming vector DNA or using selection markers.

A detectable signal may be generated in any one of a number of ways, depending on the nature of the reporter gene employed in the method of the embodiments. For example, the detectable signal may be a fluorescent signal, *e.g.,* GFP. Flow cytometry, for example, fluorescence-activated cell sorting (FACS), is commonly used to select for detectable signals based on reporter gene expression. Luciferase may also be used as a basis for an assay. Enzyme-based assays are conducted in a manner similar to a luciferase-based assay, except that the detection is not necessarily via luminescence. The detection technique will depend on the enzyme, and may therefore be optical (such as in the case of β-galactosidase). Physical and biochemical methods may also be used to identify or quantify expression of the reporter genes of the embodiments. These methods are well known to those of skill in the art.

After reprogramming of somatic cells to iPSCs, it may be desirable to substantially purify or separate one or more sub-population of cells from the cell population. Methods for separation of cells using flow cytometry, such as FACS, or magnetic activated cell sorting may be used to separate iPS cells from a heterogeneous cell population. Exemplary cell separation protocols are also shown in the examples below.

### E. Culturing of Reprogrammed Cells

Pluripotent cells may be cultured and maintained in an undifferentiated state using a variety of methods. In certain embodiments, pluripotent cells may be cultured and maintained in an essentially undifferentiated state using defined, feeder-independent culture system, such as in a TeSR or E8 medium (see, *e.g.,* Chen *et al.* (2011)). TeSR media, for example, are defined media that may be used to culture undifferentiated human embryonic stem cells. TeSR media includes both TeSR1 media and mTeSR media. TeSR includes bFGF, LiCl, γ-aminobutyric acid (GABA), pipecolic acid and TGFβ, and various methods utilizing TeSR have been described previously, *e.g.,* in U.S. Patent No. 7,449,334 and Ludwig *et al.* (2006a; 2006b). Alternately, undefined conditions may be used; for example, pluripotent cells may be cultured on fibroblast feeder cells or a medium which has been exposed to fibroblast feeder cells in order to maintain the stem cells in an undifferentiated state.

Feeder-independent culture systems and media may be used to culture and maintain pluripotent cells, such as hESC or iPSC. These approaches allow human embryonic stem cells to remain in an essentially undifferentiated state without the need for mouse fibroblast "feeder layers." As described herein, various modifications may be made to these methods in order to reduce costs as desired.

In some aspects, a matrix component may be included in a defined media for culturing and maintaining pluripotent cells in a substantially or essentially undifferentiated state. When cultured in a suitable semi-solid matrix, individual progenitors called colony-forming cells (CFCs) can proliferate to form discrete cell clusters or colonies. CFC assays may be performed by placing a cell suspension into a semi-solid medium, such as methylcellulose or collagen supplemented with nutrients and cytokines, followed by incubation, *e.g*., at about 37°C.

Various matrix components may be used to culture and maintain pluripotent cells, such as hESC or iPSC. For example, collagen IV, fibronectin, laminin, and vitronectin in combination may be used to provide a solid support for embryonic cell culturing and maintenance, as described in Ludwig *et al.* (2006a).

Matrigel™ may be used to provide a substrate for cell culture and maintenance of pluripotent cells. Matrigel™ is a gelatinous protein mixture secreted by mouse tumor cells and is commercially available from BD Biosciences (New Jersey, USA). The mixture resembles the complex extracellular environment found in many tissues and is used by cell biologists as a substrate for cell culture. Methods for human embryonic stem cell culture and maintenance in defined media with Matrigel™ are described, *e.g*., in Ludwig *et al.* (2006a), and may be used to culture pluripotent cells. It is appreciated that additional methods for the culture and maintenance of human embryonic stem cells, as would be known to one of skill, may be used with the present embodiments.

### F. Differentiation of Reprogrammed Cells

"Differentiation" is a process by which a less specialized cell becomes a more specialized cell, either in culture or *in vivo,* than it would have under the same conditions without differentiation. Under certain conditions, the proportion of progeny formed from the less specialized cell with characteristics of the more specialized cell may be at least about 1%, 5%, 25% or more in order of increasing preference.

Various approaches may be used according to the embodiments to differentiate iPS cells into cell lineages including, but not limited to, hematopoietic stem cells, immune effector cells (*e.g.,* T cells, NK cells, iNKT cells), myocytes (*e.g.,* cardiomyocytes), neurons, beta islet cells, kidney cells, lung cells, fibroblasts and epidermal cells, and tissues or organs derived therefrom.

### 1. Hematopoietic Stem Cells

iPSCs may be cultured and/or differentiated into hematopoietic progenitor cells, or hematopoietic stem cells. A skilled artisan will recognize, however, that a variety of methods may be used for generating hematopoietic progenitor cells from pluripotent cells. For example, U.S. Patent No. 8,372,642, details a highly efficient culture system to generate hematopoietic progenitor cells.

Hematopoietic progenitor cells may be generated using defined culture conditions and with the use of feeder cells. For example, after partially, essentially, or completely dissociating or individualizing pluripotent cells, the cells may be further cultured in a defined media to promote hematopoietic differentiation. It has been found that specific combinations of growth factors can substantially promote differentiation of the pluripotent cells into hematopoietic precursors and hematopoietic cell lineages. Sequential application of specific combinations of growth factors may be used to further promote differentiation of pluripotent cells. In certain embodiments, specific combinations of growth factors are critical for the hematopoietic differentiation of pluripotent cells. For example, combinations of BMP4, VEGF, Flt3 ligand, IL-3, and GMCSF may be used to promote hematopoietic differentiation. In certain aspects, sequential exposure of cell cultures to a first media that includes BMP4 and VEGF (and optionally FGF-2), followed by culture in a second media that includes Flt3 ligand, IL-3, and GMCSF can increase the differentiation of pluripotent cells into hematopoietic precursor cells and hematopoietic cells.

While differentiation of pluripotent cells into hematopoietic progenitor cells may be performed using defined or undefined conditions, it will be appreciated that defined conditions are generally preferable in embodiments where the resulting cells are intended to be administered to a human subject. Hematopoietic stem cells may be cultured from pluripotent stem cells under defined conditions (*e.g*., using a TeSR media and a matrix component such as Matrigel™ or in an E8 media).

For example, a defined medium may be used to induce hematopoietic CD34⁺ differentiation. As detailed above, the defined medium may contain the growth factors BMP-4, VEGF, Flt3 ligand, IL-3 and/or GMCSF. Substantially hypoxic conditions (*e.g.*, less than 20% O₂) may further promote hematopoietic or endothelial differentiation.

Although the use of defined methods for the differentiation of pluripotent cells into hematopoietic precursor cells may be preferred in certain instances, undefined approaches may nonetheless be used in various embodiments. One undefined method for the differentiation of hematopoietic stem cells from iPSCs involves culturing the iPSCs on feeder cells, such as a mouse embryonic fibroblast (MEF) feeder layer or the mouse stromal cell line OP9, which induces robust differentiation to CD34⁺. In contrast to defined conditions, use of OP9 cells generally does not require extra growth factors to induce CD34⁺ differentiation.

### 2. Hematopoietic Cells

Pluripotent stem cells, such as iPSCs, are capable of unlimited self-renewal while retaining the ability to derive myeloid, erythroid, and megakaryocytic hematopoietic cell lineages when induced or supported to differentiate under appropriate conditions.

Exemplary methods of iPS cell differentiation into hematopoietic cells may include, for example, methods disclosed by U.S. Patent Nos. 8,557,580 and 8,372,642 and WO 12/109208, or other methods (Chadwick *et al.,* 2003; Ng *et al.,* 2005). Fibronectin differentiation methods may also be used for blood lineage differentiation, as exemplified in Wang *et al.* (2007). Hematopoietic cells can also be made by culturing stem cells with hematogenic cytokines and a bone morphogenic protein, as described in U.S. Patent Publn. 2003/0153082.

### a. Chimeric Antigen Receptors and T-cell Receptors

As used herein, the term "antigen" is a molecule capable of being bound by an antibody or T-cell receptor. An antigen is additionally capable of inducing a humoral immune response and/or cellular immune response leading to the production of B and/or T lymphocytes. The terms "tumor-associated antigen" and "cancer cell antigen" are used interchangeably herein. In each case, the terms refer to proteins, glycoproteins or carbohydrates that are specifically or preferentially expressed by cancer cells.

The term "chimeric antigen receptors (CARs)," as used herein, may refer to artificial T-cell receptors, chimeric T-cell receptors, or chimeric immunoreceptors, for example, and encompass engineered receptors that graft an artificial specificity onto a particular immune effector cell. CARs may be employed to impart the specificity of a monoclonal antibody onto a T cell, thereby allowing a large number of specific T cells to be generated, for example, for use in adoptive cell therapy. In specific embodiments, CARs direct specificity of the cell to a tumor associated antigen, for example. In some embodiments, CARs comprise an intracellular activation domain, a transmembrane domain, and an extracellular domain comprising a tumor associated antigen binding region. In particular aspects, CARs comprise fusions of single-chain variable fragments (scFv) derived from monoclonal antibodies, fused to CD3-zeta transmembrane domain and endodomain. The specificity of other CAR designs may be derived from ligands of receptors (*e.g*., peptides) or from pattern-recognition receptors, such as Dectins. For example, one can target malignant B cells by redirecting the specificity of T cells by using a CAR specific for the B-lineage molecule, CD19. In certain cases, the spacing of the antigen-recognition domain can be modified to reduce activation-induced cell death. In certain cases, CARs comprise domains for additional co-stimulatory signaling, such as CD3-zeta, FcR, CD27, CD28, CD137, DAP10, and/or OX40. In some cases, molecules can be co-expressed with the CAR, including co-stimulatory molecules, reporter genes for imaging (*e.g*., for positron emission tomography), gene products that conditionally ablate the T cells upon addition of a pro-drug, homing receptors, chemokines, chemokine receptors, cytokines, and cytokine receptors.

Embodiments of the present disclosure involve nucleic acids, including nucleic acids encoding an antigen-specific chimeric antigen receptor (CAR) polypeptide, including a CAR that has been humanized to reduce immunogenicity (hCAR), comprising an intracellular signaling domain, a transmembrane domain, and an extracellular domain comprising one or more signaling motifs. In certain embodiments, the CAR may recognize an epitope comprised of the shared space between one or more antigens. Pattern recognition receptors, such as Dectin-1, may be used to derive specificity to a carbohydrate antigen. In certain embodiments, the binding region can comprise complementary determining regions of a monoclonal antibody, variable regions of a monoclonal antibody, and/or antigen binding fragments thereof. In another embodiment, that specificity is derived from a peptide (*e.g*., cytokine) that binds to a receptor. A complementarity determining region (CDR) is a short amino acid sequence found in the variable domains of antigen receptor (*e.g*., immunoglobulin and T-ccll receptor) proteins that complements an antigen and therefore provides the receptor with specificity for that particular antigen. Each polypeptide chain of an antigen receptor contains three CDRs (CDR1, CDR2, and CDR3). Since antigen receptors are typically composed of two polypeptide chains, there are six CDRs for each antigen receptor that can come into contact with the antigen -- each heavy and light chain contains three CDRs. Because most sequence variation associated with immunoglobulins and T-cell receptors are found in the CDRs, these regions are sometimes referred to as hypervariable domains. Among these, CDR3 shows the greatest variability as it is encoded by a recombination of the VJ (VDJ in the case of heavy chain and TCR αβ chain) regions.

The term "T-cell receptor (TCR)" as used herein refers to a protein receptor on T cells that is composed of a heterodimer of an alpha (α) and beta (β) chain, although in some cells the TCR consists of gamma and delta (γ/δ) chains. In embodiments of the disclosure, the TCR may be modified on any cell comprising a TCR, including a helper T cell, a cytotoxic T cell, a memory T cell, regulatory T cell, natural killer T cell, and gamma delta T cell, for example. "Chimeric TCR" means a receptor that comprises intracellular signaling, transmembrane, and extracellular domains, where the extracellular domain is capable of specifically binding in an MHC unrestricted manner an antigen that is not normally bound by a T-cell receptor in that manner. Stimulation of the T cells by the antigen under proper conditions results in proliferation (expansion) of the cells and/or production of IL-2. The method is applicable to transfection with chimeric TCRs that are specific for target antigens, such as chimeric TCRs that are specific for HER2/Neu, ERBB2, folate binding protein, renal cell carcinoma, and HIV-1 envelope glycoproteins gp120 and gp41. Other cell-surface target antigens include, but are not limited to, CD20, carcinoembryonic antigen, mesothelin, ROR1, c-Met, CD56, GD2, GD3, alphafetoprotein, CD23, CD30, CD123, IL-11Ralpha, kappa chain, lambda chain, CD70, CA-125, MUC-1, EGFR and variants, epithelial tumor antigen, and so forth.

It is contemplated that humanized CAR nucleic acids are derived from human genes to enhance cellular immunotherapy for human patients. In a specific embodiment, the disclosure includes a full-length CAR cDNA or coding region. The antigen binding regions or domain can comprise a fragment of the V_{H} and V_{L} chains of a single-chain variable fragment (scFv) derived from a particular human monoclonal antibody, such as those described in U.S. Patent No. 7,109,304. The fragment can also be any number of different antigen binding domains of a human antigen-specific antibody. In a more specific embodiment, the fragment is an antigen-specific scFv encoded by a sequence that is optimized for human codon usage for expression in human cells.

The arrangement could be multimeric, such as a diabody or multimers. The multimers are most likely formed by cross pairing of the variable portion of the light and heavy chains into a diabody. The hinge portion of the construct can have multiple alternatives from being totally deleted, to having the first cysteine maintained, to a proline rather than a serine substitution, to being truncated up to the first cysteine. The Fc portion can be deleted. Any protein that is stable and/or dimerizes can serve this purpose. One could use just one of the Fc domains, *e.g*., either the CH2 or CH3 domain from human immunoglobulin. One could also use the hinge, CH2 and CH3 region of a human immunoglobulin that has been modified to improve dimerization. One could also use just the hinge portion of an immunoglobulin. One could also use portions of CD8alpha.

The intracellular signaling domain (which may be referred to as the cytoplasmic domain) of the chimeric receptor of the embodiments is responsible for activation of at least one of the normal effector functions of the immune cell in which the chimeric receptor has been placed. The term "effector function" refers to a specialized function of a differentiated cell. Effector function of a T cell, for example, may be cytolytic activity or helper activity, such as the secretion of cytokines. Effector function in a naive, memory, or memory-type T cell includes antigen-dependent proliferation. Thus, the term "intracellular signaling domain" refers to the portion of a protein that transduces the effector function signal and directs the cell to perform a specialized function. While usually the entire intracellular signaling domain will be employed, in many cases it will not be necessary to use the entire intracellular polypeptide. To the extent that a truncated portion of the intracellular signaling domain still transduces the effector function signal, such truncated portion may be used in place of the intact chain. The term intracellular signaling domain is thus meant to include any truncated portion of the intracellular signaling domain sufficient to transduce the effector function signal. Examples include the zeta chain of the T-cell receptor or any of its homologs (*e.g*., eta, delta, gamma, or epsilon), MB1 chain, B29, Fc RIII, Fc RI, and combinations of all or part of one or more signaling molecules, such as CD3ζ and CD28, CD27, 4-1BB (CD137), DAP10, DAP12, OX40 (CD134), ICOS/CD278, IL-2Rbeta/CD122, IL-2Ralpha/CD132, CD40, and combinations thereof, as well as other similar molecules and fragments. Intracellular signaling portions of other members of the families of activating proteins can be used, such as FcγRIII and FcεRI. In some embodiments, one employs any part of the endogenous T cell receptor complex in the intracellular domain. One or multiple cytoplasmic domains may be employed, as so-called third generation CARs have at least two or three signaling domains fused together for additive or synergistic effect, for example.

The antigen-specific extracellular domain and the intracellular signaling-domain may be linked by a transmembrane domain, such as the human IgG₄Fc hinge and Fc regions. Alternatives include the human CD4 transmembrane domain, the human CD28 transmembrane domain, the transmembrane human CD3ζ domain, or a cysteine mutated human CD3ζ domain, or other transmembrane domains from other human transmembrane signaling proteins, such as CD16 and CD8 and erythropoietin receptor.

In some embodiments, the CAR nucleic acid comprises a sequence encoding other costimulatory receptors, such as a transmembrane domain and a modified CD28 intracellular signaling domain. Other costimulatory receptors include, but are not limited to, one or more of CD28, CD27, OX40 (CD134), DAP10, and 4-1BB (CD137). An additional signal provided by a human costimulatory receptor inserted in a human CAR is important for full activation of T cells and could help improve *in vivo* persistence and the therapeutic success of adoptive immunotherapy.

In particular embodiments, the disclosure concerns isolated nucleic acid segments and expression cassettes incorporating DNA sequences that encode the CAR. Vectors of the embodiments are designed, primarily, to deliver desired genes to cells under the control of regulated eukaryotic promoters, for example, MNDU3 promoter, CMV promoter, EF1alpha promoter, or Ubiquitin promoter. Also, the vectors may contain a selectable marker, if for no other reason, to facilitate their manipulation *in vitro.* In other embodiments, the CAR can be expressed from mRNA *in vitro* transcribed from a DNA template.

Chimeric antigen receptor molecules are recombinant and are distinguished by their ability to both bind antigen and transduce activation signals *via* immunoreceptor activation motifs (ITAM's) present in their cytoplasmic tails. Receptor constructs utilizing an antigen-binding moiety (for example, generated from single chain antibodies (scFv)) afford the additional advantage of being "universal" in that they bind native antigen on the target cell surface in an HLA-independent fashion. Re-directed T cell effector mechanisms include tumor recognition and lysis by CTL.

In some embodiments, the chimeric antigen receptor comprises: a) an intracellular signaling domain, b) a transmembrane domain, and c) an extracellular domain comprising an antigen binding region. The use of human antibodies as the source of antigen binding regions is preferred. The use of human sequences in the CAR can avoid immune-mediated recognition and therefore elimination by endogenous T cells that reside in the recipient and recognize processed antigen in the context of HLA.

In certain embodiments of the chimeric antigen receptor, the antigen-specific portion of the receptor (which may be referred to as an extracellular domain comprising an antigen binding region) comprises a tumor associated antigen or a pathogen-specific antigen binding domain, optionally including a carbohydrate antigen recognized by pattern-recognition receptors, such as Dectin-1.

A tumor-associated antigen may be of any kind, such as those expressed on the cell surface of tumor cells. Exemplary embodiments of tumor associated antigens include CD19, CD20, carcinoembryonic antigen, alphafetoprotein, CA-125, MUC-1, CD56, EGFR, c-Met, AKT, Her2, Her3, epithelial tumor antigen, melanoma-associated antigen, mutated p53, mutated ras, and so forth. In certain embodiments, the CAR can be co-expressed with a membrane-bound cytokine to improve persistence when there is a low amount of tumor-associated antigen. For example, CAR can be co-expressed with membrane-bound IL-15.

In certain embodiments intracellular tumor associated antigens may be targeted, such as HA-1, survivin, WT1, and p53. This can be achieved by a CAR that recognizes the processed peptide described from the intracellular tumor associated antigen in the context of HLA. In addition, a universal T cell may be genetically modified to express a T-cell receptor pairing that recognizes the intracellular processed tumor associated antigen in the context of HLA.

The pathogen may be of any kind, but in specific embodiments the pathogen is a fungus, bacteria, or virus, for example. Exemplary viral pathogens include those of the families of Adenoviridae, Epstein-Barr virus (EBV), Cytomegalovirus (CMV), Respiratory Syncytial Virus (RSV), JC virus, BK virus, HSV, HHV family of viruses, Picornaviridae, Herpesviridae, Hepadnaviridae, Flaviviridae, Retroviridae, Orthomyxoviridae, Paramyxoviridae, Papovaviridae, Polyomavirus, Rhabdoviridae, and Togaviridae. Exemplary pathogenic viruses cause smallpox, influenza, mumps, measles, chickenpox, ebola, and rubella. Exemplary pathogenic fungi include *Candida*, *Aspergillus*, *Cryptococcus, Histoplasma*, *Pneumocystis,* and *Stachybotrys.* Exemplary pathogenic bacteria include *Streptococcus, Pseudomonas, Shigella, Campylobacter, Staphylococcus, Helicobacter, E. coli, Rickettsia, Bacillus, Bordetella, Chlamydia, Spirochetes,* and *Salmonella.* In one embodiment the pathogen receptor Dectin-1 can be used to generate a CAR that recognizes the carbohydrate structure on the cell wall of fungi. T cells genetically modified to express the CAR based on the specificity of Dectin-1 can recognize *Aspergillus* and target hyphal growth. In another embodiment, CARs can be made based on an antibody recognizing viral determinants (*e.g*., the glycoproteins from CMV and Ebola) to interrupt viral infections and pathology.

A chimeric antigen receptor according to the embodiments can be produced by any means known in the art, though preferably it is produced using recombinant DNA techniques. A nucleic acid sequence encoding the several regions of the chimeric receptor can be prepared and assembled into a complete coding sequence by standard techniques of molecular cloning (genomic library screening, PCR, primer-assisted ligation, scFv libraries from yeast and bacteria, site-directed mutagenesis, *etc.*)*.* The resulting coding region can be inserted into an expression vector and used to transform a cell of interest, for example, an iPSC.

### b. Antigen Presenting Cells

In some cases, APCs are useful in preparing therapeutic compositions and cell therapy products of the embodiments. For general guidance regarding the preparation and use of antigen-presenting systems, such as system using artificial APCs, see, *e.g.,* U.S. Patent Nos. 6,225,042, 6,355,479, 6,362,001, 6,790,662, 7,993,638, and 8,124,408; and U.S. Patent Publn. No. 2009/0004142.

APCs are typically incubated with a peptide of an optimal length that allows for direct binding of the peptide to the MHC molecule without additional processing. Alternatively, the cells can express and antigen of interest (*i.e.,* in the case of MHC-independent antigen recognition). In addition to peptide-MHC molecules or antigens of interest, the APC systems may also comprise at least one exogenous assisting molecule. Any suitable number and combination of assisting molecules may be employed. The assisting molecule may be selected from assisting molecules such as co-stimulatory molecules and adhesion molecules. Exemplary co-stimulatory molecules include CD86 and B7.1 (B7.1 was previously known as B7 and also known as CD80), which among other things, bind to CD28 and/or CTLA-4 molecules on the surface of T cells, thereby affecting, for example, T-cell expansion, Th1 differentiation, short-term T-cell survival, and cytokine secretion such as interleukin (IL)-2 (see, Kim *et al.,* 2004). Adhesion molecules may include carbohydrate-binding glycoproteins such as selectins, transmembrane binding glycoproteins such as integrins, calcium-dependent proteins such as cadherins, and single-pass transmembrane immunoglobulin (Ig) superfamily proteins, such as intercellular adhesion molecules (ICAMs), that promote, for example, cell-to-cell or cell-to-matrix contact. Exemplary adhesion molecules include LFA-3 and ICAMs, such as ICAM-1. Techniques, methods, and reagents useful for selection, cloning, preparation, and expression of exemplary assisting molecules, including co-stimulatory molecules and adhesion molecules, are exemplified in, *e.g*., U.S. Patent Nos. 6,225,042, 6,355,479, and 6,362,001.

Cells selected to become artificial APCs, preferably have deficiencies in intracellular antigen-processing, intracellular peptide trafficking, and/or intracellular MHC Class I or Class II molecule-peptide loading, or are poikilothermic (*i.e.,* less sensitive to temperature challenge than mammalian cell lines), or possess both deficiencies and poikilothermic properties. In some aspects, cells selected to become APCs also lack the ability to express at least one endogenous counterpart (*e.g*., endogenous MHC Class I or Class II molecule and/or endogenous assisting molecules as described above) to the exogenous MHC Class I or Class II molecule and assisting molecule components that are introduced into the cells. Furthermore, artificial APCs, in some cases, retain the deficiencies and poikilothermic properties that were possessed by the cells prior to their modification to generate the aAPCs. Exemplary aAPCs either constitute or are derived from a transporter associated with antigen processing (TAP)-deficient cell line, such as an insect cell line. An exemplary poikilothermic insect cells line is a Drosophila cell line, such as a Schneider 2 cell line (see, *e.g*., Schneider, 1972). Illustrative methods for the preparation, growth, and culture of Schneider 2 cells, are provided in U.S. Patent Nos. 6,225,042, 6,355,479, and 6,362,001.

In one embodiment, APCs are also subjected to a freeze-thaw cycle. In an exemplary freeze-thaw cycle, the APCs may be frozen by contacting a suitable receptacle containing the APCs with an appropriate amount of liquid nitrogen, solid carbon dioxide (*i.e*., dry ice), or similar low-temperature material, such that freezing occurs rapidly. The frozen APCs are then thawed, either by removal of the APCs from the low-temperature material and exposure to ambient room temperature conditions, or by a facilitated thawing process in which a lukewarm water bath or warm hand is employed to facilitate a shorter thawing time. Additionally, APCs may be frozen and stored for an extended period of time prior to thawing. Frozen APCs may also be thawed and then lyophilized before further use. Preferably, preservatives that might detrimentally impact the freeze-thaw procedures, such as dimethyl sulfoxide (DMSO), polyethylene glycols (PEGs), and other preservatives, are absent from media containing APCs that undergo the freeze-thaw cycle, or are essentially removed, such as by transfer of APCs to media that is essentially devoid of such preservatives.

In other certain embodiments, xenogenic nucleic acid and nucleic acid endogenous to aAPCs, may be inactivated by crosslinking, so that essentially no cell growth, replication or expression of nucleic acid occurs after the inactivation. In one embodiment, aAPCs are inactivated at a point subsequent to the expression of exogenous MHC and assisting molecules, presentation of such molecules on the surface of the aAPCs, and loading of presented MHC molecules with selected peptide or peptides. Accordingly, such inactivated and selected peptide loaded aAPCs, while rendered essentially incapable of proliferating or replicating, retain selected peptide presentation function. Preferably, the crosslinking also yields aAPCS that are essentially free of contaminating microorganisms, such as bacteria and viruses, without substantially decreasing the antigen-presenting cell function of the aAPCs. Thus crosslinking maintains the important APC functions of aAPCs while helping to alleviate concerns about safety of a cell therapy product developed using the aAPCs. For methods related to crosslinking and aAPCs, see for example, U.S. Patent No. 8,124,408.

### 3. Liver Cells

Hepatocytes can be differentiated from pluripotent stem cells using an inhibitor of histone deacetylase, as described in U.S. Patent Nos. 6,458,589 and 6,506,574. Staged protocols for differentiating pluripotent stem cells into hepatocytes are described in U.S. Patent No. 7,473,555. Hepatocytes in certain aspects of this invention can be made by culturing pluripotent stem cells in media to direct the differentiation into hepatocytes as described in U.S. Patent No. 8,283,168. Hepatocytes in certain aspects of this invention can be made by culturing pluripotent stem cells or other non-hepatocytes in a medium under conditions that increase the intracellular level of hepatocyte programming factors to be sufficient to promote programming of the cells into hepatocytes.

### 4. Nerve Cells

Neural cells can be generated from pluripotent stem cells according to the method described in U.S. Patent Nos. 6,833,269 and 7,250,294; U.S. Patent Publn. No. 2012/0276063; and Carpenter *et al.,* 2001. Oligodendrocytes can be generated from pluripotent stem cells, see U.S. Patent No. 7,285,415 and Keirstead *et al.,* 2005. Derivation of retinal pigment epithelial cells has also been reported (Klimanskaya *et al.,* 2004).

### 5. Heart Cells

The iPS cells provided herein may be differentiated into cardiomyocytes according to methods described in U.S. Patent No. 8,415,155. Cardiomyocytes or cardiomyocyte precursors can be generated from pluripotent stem cells such as iPS cells according to the method provided in U.S. Patent No. 7,763,464. Exemplary methods of cardiac differentiation of iPS cells may include embryoid body (EB) methods (Zhang *et al.,* 2009), OP9 stroma cell methods (Narazaki *et al.,* 2008), or growth factor/chemical methods (see U.S. Patent Nos. 7,897,389; 7,955,849; and 8,951,798).

### 6. Pancreatic Cells

Islet cells can be differentiated from pluripotent stem cells (WO 03/050249).

### 7. Other Cell Types

Mesenchymal progenitors and fibroblasts can be generated from pluripotent stem cells according to the method described in WO 03/004605. Stem cell-derived mesenchymal cells can then be further differentiated into osteoblast lineage cells in a medium containing an osteogenic factor, such as bone morphogenic protein (particularly BMP4), a ligand for a human TGF-β receptor, or a ligand for a human vitamin D receptor (WO 03/004605).

### G. Bioreactors and Automation

One or more steps for the culture of stem cells, and/or cell differentiated therefrom, may be automated. Automating a process using robotic or other automation can allow for more efficient and economical methods for the production, culture, and differentiation of cells. For example, robotic automation may be utilized in conjunction with one or more of the culture of induced pluripotent stem cells, passaging, addition of media, addition of differentiation media, culture in differentiation media, and separation of cell types, *e.g*., using magnetic separation or FACS.

A bioreactor may also be used in conjunction with the present embodiments to culture, maintain, and/or differentiate cells according to the present embodiments. Bioreactors provide the advantage of allowing for the "scaling up" of a process in order to produce an increased amount of cells. Various bioreactors may be used with the present embodiments, including batch bioreactors, fed batch bioreactors, continuous bioreactors (*e.g*., a continuous stirred-tank reactor model), and/or a chemostat. For example, spinner flasks may be used to scale up methods for the maintenance and/or differentiation of pluripotent cells to allow for the generation of increased numbers of cells.

Robotic automation specifically envisioned for use with the present embodiments may be obtained from, for example, Tecan (CA, USA). Robotics may include liquid handling tools such as cap-piercing probes and disposable tips to minimize carry-over between samples. In various embodiments, robotics may be utilized in conjunction with one or more bioreactor for culturing cells (*e.g*., during the maintenance or growth of iPSCs, the differentiation of iPSCs into hematopoietic cells, or the differentiation of hematopoietic cells into subsequent lineages such as erythrocytes, *etc.*)*.*

In certain embodiments, it may be useful to miniaturize or "scale down" methods of the present embodiments. These approaches may be particularly useful, *e.g.,* where the methods comprise a high-throughput screen. High-throughput screens may be used to evaluate one or more property of a chimeric antigen receptor. Miniaturization of the methods may involve the use of low-attachment plates (*e.g.,* 96 well plates) and/or culture of cells under low oxygen (*e.g*., less than about 25% O₂ or about 5% O₂) conditions.

Methods of the present embodiments may be utilized in a single cell assay, using robotic automation, by including the ROCK inhibitors HA100 and/or H1152 in the media to induce cells to adhere as single cells to a plate. The addition of the small molecules HA100 or H1152 or Y-27632 to the culture system can greatly improve the viability of pluripotent cells, including iPSCs. In certain embodiments, survival of pluripotent cells in a TeSR media can be improved by the inclusion of a ROCK inhibitor or a PKC inhibitor, particularly after the cells are proteolytically or mechanically separated into clumps or individualized. The ROCK inhibitors can promote individualized iPS cells to attach to a surface and grow. Some or all of the process of maintenance or proliferation of pluripotent cells, as well as differentiation into hematopoietic precursor cells or a specific hematopoietic lineage may be automated. Part of all of the automated methods may utilize defined conditions.

### III. Methods of Treating

### A. Immune System and Immunotherapy

In some embodiments, a medical disorder is treated by transfer of an immune effector cell (*e.g.,* a T cell) that elicits a specific immune response. Thus, a basic understanding of the immunologic response is necessary.

The cells of the adaptive immune system are a type of leukocyte, called a lymphocyte. B cells and T cells are the major types of lymphocytes. B cells and T cells are derived from the same pluripotent hematopoietic stem cells, and are indistinguishable from one another until after they are differentiated. B cells play a large role in the humoral immune response, whereas T cells are intimately involved in cell-mediated immune responses. T cells can be distinguished from other lymphocyte types, such as B cells and NK cells, by the presence of a special receptor on their cell surface called the T-cell receptor (TCR). In nearly all other vertebrates, B cells and T cells are produced by stem cells in the bone marrow. T cells travel to and develop in the thymus, from which they derive their name. In humans, approximately 1%-2% of the lymphocyte pool recirculates each hour to optimize the opportunities for antigen-specific lymphocytes to find their specific antigen within the secondary lymphoid tissues.

T lymphocytes arise from hematopoietic stem cells in the bone marrow, and typically migrate to the thymus gland to mature. T cells express a unique antigen binding receptor on their membrane (T-cell receptor), which can only recognize antigen in association with major histocompatibility complex (MHC) molecules on the surface of other cells. There are at least two populations of T cells, known as T helper cells and T cytotoxic cells. T helper cells and T cytotoxic cells are primarily distinguished by their display of the membrane bound glycoproteins CD4 and CD8, respectively. T helper cells secret various lymphokines that are crucial for the activation of B cells, T cytotoxic cells, macrophages, and other cells of the immune system. In contrast, T cytotoxic cells that recognize an antigen-MHC complex proliferate and differentiate into effector cell called cytotoxic T lymphocytes (CTLs). CTLs eliminate cells of the body displaying antigen, such as virus infected cells and tumor cells, by producing substances that result in cell lysis. Natural killer cells (or NK cells) are a type of cytotoxic lymphocyte that constitutes a major component of the innate immune system. NK cells play a major role in the rejection of tumors and cells infected by viruses. The cells kill by releasing small cytoplasmic granules of proteins called perforin and granzyme that cause the target cell to die by apoptosis.

Antigen-presenting cells, which include macrophages, B lymphocytes, and dendritic cells, are distinguished by their expression of a particular MHC molecule. APCs internalize antigen and re-express a part of that antigen, together with the MHC molecule on their outer cell membrane. The major histocompatibility complex (MHC) is a large genetic complex with multiple loci. The MHC loci encode two major classes of MHC membrane molecules, referred to as class I and class II MHCs. T helper lymphocytes generally recognize antigen associated with MHC class II molecules, and T cytotoxic lymphocytes recognize antigen associated with MHC class I molecules. In humans the MHC is referred to as the HLA complex and in mice the H-2 complex.

The T-cell receptor, or TCR, is a molecule found on the surface of T lymphocytes (or T cells) that is generally responsible for recognizing antigens bound to major histocompatibility complex (MHC) molecules. It is a heterodimer consisting of an alpha and beta chain in 95% of T cells, while 5% of T cells have TCRs consisting of gamma and delta chains. Engagement of the TCR with antigen and MHC results in activation of its T lymphocyte through a series of biochemical events mediated by associated enzymes, co-receptors, and specialized accessory molecules.

Autoimmune disease, or autoimmunity, is the failure of an organism to recognize its own constituent parts (down to the sub-molecular levels) as "self," which results in an immune response against its own cells and tissues. Any disease that results from such an aberrant immune response is termed an autoimmune disease.

### B. Growth and Administration of Cells

In certain aspects, cells are differentiated from iPSCs. In one aspect, T cells differentiated from iPSCs may be made recombinant through the introduction of a CAR or TCR by, for example, gene transfer. In certain aspects, the cells may be propagated for days, weeks, or months *ex vivo* as a bulk population within about 1, 2, 3, 4, 5 days or more following gene transfer. In a further aspect, the recombinant T cells may be cloned and a clone demonstrating presence of a single integrated or episomally maintained expression cassette or plasmid, and expression of the chimeric receptor, is expanded *ex vivo.* The recombinant T cells may be expanded by stimulation with IL-2, or other cytokines that bind the common gamma-chain (*e.g*., IL-7, IL-12, IL-15, IL-21, and others). The recombinant T cells may be expanded by stimulation with artificial antigen presenting cells. The recombinant T cells may be expanded on artificial antigen presenting cell or with an antibody, such as OKT3, which cross links CD3 on the T-cell surface. In a further aspect, the genetically modified cells may be cryopreserved.

In certain embodiments of the disclosure, the CAR⁺ cells are delivered to an individual in need thereof, such as an individual that has cancer, an autoimmune disorder, or an infection. The cells then enhance the individual's immune system to attack the respective cancer or pathogenic cells. In some cases, the individual is provided with one or more doses of the antigen-specific CAR⁺ T-cells. In cases where the individual is provided with two or more doses of the antigen-specific CAR⁺ T-cells, the duration between the administrations should be sufficient to allow time for propagation in the individual, and in specific embodiments the duration between doses is 1, 2, 3, 4, 5, 6, 7, or more days.

Suitable doses of immune effector cells for a therapeutic effect would be at least 10⁵ or between about 10⁵ and about 10¹⁰ cells per dose, for example, preferably in a series of dosing cycles. An exemplary dosing regimen consists of four one-week dosing cycles of escalating doses, starting at least at about 10⁵ cells on Day 0, for example increasing incrementally up to a target dose of about 10¹⁰ cells within several weeks of initiating an intra-patient dose escalation scheme. Suitable modes of administration include intravenous, subcutaneous, intracavitary (for example by reservoir-access device), intraperitoneal, and direct injection into a tumor mass.

A pharmaceutical composition in accordance with the embodiments can be used alone or in combination with other well-established agents useful for treating cancer or infectious disease. Whether delivered alone or in combination with other agents, the pharmaceutical composition of the embodiments can be delivered via various routes and to various sites in a mammalian, particularly human, body to achieve a particular effect. One skilled in the art will recognize that, although more than one route can be used for administration, a particular route can provide a more immediate and more effective reaction than another route. For example, intradermal delivery may be advantageously used over inhalation for the treatment of melanoma. Local or systemic delivery can be accomplished by administration comprising application or instillation of the formulation into body cavities, inhalation or insufflation of an aerosol, or by parenteral introduction, comprising intramuscular, intravenous, intraportal, intrahepatic, peritoneal, subcutaneous, or intradermal administration.

A composition of the embodiments can be provided in unit dosage form wherein each dosage unit, *e.g.,* an injection, contains a predetermined amount of the composition, alone or in appropriate combination with other active agents. The term unit dosage form as used herein refers to physically discrete units suitable as unitary dosages for human and animal subjects, each unit containing a predetermined quantity of the composition of the embodiments, alone or in combination with other active agents, calculated in an amount sufficient to produce the desired effect, in association with a pharmaceutically acceptable diluent, carrier, or vehicle, where appropriate. The specifications for the novel unit dosage forms of the embodiments depend on the particular pharmacodynamics associated with the pharmaceutical composition in the particular subject.

Desirably an effective amount or sufficient number of the cells is present in the composition and introduced into the subject such that long-term, specific, antitumor responses are established to reduce the size of a tumor or eliminate tumor growth or regrowth than would otherwise result in the absence of such treatment. Desirably, the amount of cells reintroduced into the subject causes a 10%, 20%, 30%, 40%, 50%, 60%, 70%, 80%, 90%, 95%, 98%, or 100% decrease in tumor size when compared to otherwise same conditions wherein the cells are not present.

Accordingly, the amount of cells administered should take into account the route of administration and should be such that a sufficient number of the cells will be introduced so as to achieve the desired therapeutic response. Furthermore, the amounts of each active agent included in the compositions described herein (*e.g.,* the amount per each cell to be contacted or the amount per certain body weight) can vary in different applications. In general, the concentration of cells desirably should be sufficient to provide in the subject being treated at least from about 1 × 10⁶ to about 1 × 10⁹ transduced cells, even more desirably, from about 1 × 10⁷ to about 5 × 10⁸ transduced cells, although any suitable amount can be utilized either above, *e.g.,* greater than 5 × 10⁸ cells, or below, *e.g.,* less than 1 × 10⁷ cells. The dosing schedule can be based on well-established cell-based therapies (see, *e.g.,* Topalian and Rosenberg, 1987; U.S. Patent No. 4,690,915), or an alternate continuous infusion strategy can be employed.

These values provide general guidance of the range of cells to be utilized by the practitioner upon optimizing the method of the embodiments. The recitation herein of such ranges by no means precludes the use of a higher or lower amount of a component, as might be warranted in a particular application. For example, the actual dose and schedule can vary depending on whether the compositions are administered in combination with other pharmaceutical compositions, or depending on interindividual differences in pharmacokinetics, drug disposition, and metabolism. One skilled in the art readily can make any necessary adjustments in accordance with the exigencies of the particular situation.

### IV. Vector Construction and Delivery

A "vector" or "construct" (sometimes referred to as gene delivery or gene transfer "vehicle") refers to a macromolecule or complex of molecules comprising a polynucleotide to be delivered to a host cell, either *in vitro* or *in vivo.* A "plasmid," a type of a vector, is an extra-chromosomal DNA molecule separate from the chromosomal DNA which is capable of replicating independently of the chromosomal DNA. In certain cases, it is circular and double-stranded.

In certain embodiments, vectors, such as reprogramming vectors or CAR vectors, may be viral vectors, for example, retroviral vectors or lentiviral vectors to express these proteins in cells. In certain embodiments, vectors may be extra-chromosomally replicating vectors, which will not be integrated into the host cell genome and may be lost during generations of replication. Details of components of these vectors and delivery methods are disclosed below.

One of skill in the art would be well equipped to construct a vector through standard recombinant techniques (see, for example, Sambrook *et al.,* 2001 and Ausubel *et al.,* 1994). Vectors include, but are not limited to, plasmids, cosmids, viruses (*e.g*., bacteriophage, animal viruses, and plant viruses), artificial chromosomes (*e.g.,* YACs), retroviral vectors (*e.g.,* derived from Moloney murine leukemia virus vectors (MoMLV), MSCV, SFFV, MPSV, SNV *etc.*)*,* lentiviral vectors (*e.g*., derived from HIV-1, HIV-2, SIV, BIV, FIV *etc.*)*,* adenoviral (Ad) vectors including replication competent, replication deficient and gutless forms thereof, adeno-associated viral (AAV) vectors, simian virus 40 (SV40) vectors, bovine papilloma virus vectors, Epstein-Barr virus, herpes virus vectors, vaccinia virus vectors, Harvey murine sarcoma virus vectors, murine mammary tumor virus vectors, and Rous sarcoma virus vectors.

### A. Regulatory Elements

Vectors can also comprise other components or functionalities that further modulate gene delivery and/or gene expression, or that otherwise provide beneficial properties to the targeted cells. Such other components include, for example, components that influence binding or targeting to cells (including components that mediate cell-type or tissue-specific binding); components that influence uptake of the vector nucleic acid by the cell; components that influence localization of the polynucleotide within the cell after uptake (such as agents mediating nuclear localization); and components that influence expression of the polynucleotide.

Eukaryotic expression cassettes included in the vectors may contain (in a 5'-to-3' direction) a eukaryotic transcriptional promoter operably linked to a protein-coding sequence, splice signals including intervening sequences, and a transcriptional termination/polyadenylation sequence.

### 1. Promoter/Enhancers

A "promoter" is a control sequence that is a region of a nucleic acid sequence at which initiation and rate of transcription are controlled. The phrases "operatively positioned," "operatively linked," "under control," and "under transcriptional control" mean that a promoter is in a correct functional location and/or orientation in relation to a nucleic acid sequence to control transcriptional initiation and/or expression of that sequence. A promoter may or may not be used in conjunction with an "enhancer," which refers to a cis-acting regulatory sequence involved in the transcriptional activation of a nucleic acid sequence.

The promoters employed may be constitutive, tissue-specific, inducible, and/or useful under the appropriate conditions to direct high level expression of the introduced DNA segment, such as is advantageous in the large-scale production of recombinant proteins and/or peptides. The promoter may be heterologous or endogenous. Non-limiting examples of promoters include early or late viral promoters, such as, SV40 early or late promoters, cytomegalovirus (CMV) immediate early promoters, Rous Sarcoma Virus (RSV) early promoters; eukaryotic cell promoters such as the eukaryotic elongation factor 1α promoter; and concatenated response element promoters.

### 2. Initiation Signals

A specific initiation signal also may be required for efficient translation of coding sequences. These signals include the ATG initiation codon or adjacent sequences. Exogenous translational control signals, including the ATG initiation codon, may need to be provided. It is well known that the initiation codon must be "in-frame" with the reading frame of the desired coding sequence to ensure translation of the entire insert.

### 3. Splice Sites

Most transcribed eukaryotic RNA molecules will undergo RNA splicing to remove introns from the primary transcripts. Vectors containing genomic eukaryotic sequences may require donor and/or acceptor splicing sites to ensure proper processing of the transcript for protein expression.

### 4. Termination and Polyadenylation Signals

The vectors or constructs of the embodiments may comprise at least one termination signal. A "termination signal" or "terminator" is comprised of the DNA sequences involved in specific termination of an RNA transcript by an RNA polymerase. A terminator may be necessary *in vivo* to achieve desirable message levels. In eukaryotic systems, the terminator region may also comprise specific DNA sequences that permit site-specific cleavage of the new transcript so as to expose a polyadenylation site. Terminators contemplated for use according to the embodiments include any known terminator of transcription described herein or known to one of ordinary skill in the art, including but not limited to, for example, the termination sequences of genes, such as, for example, the bovine growth hormone terminator or viral termination sequences, such as for example the SV40 terminator.

### B. Multiple Cloning Sites

Vectors can include a multiple cloning site (MCS), which is a nucleic acid region that contains multiple restriction enzyme sites, any of which can be used in conjunction with standard recombinant technology to digest the vector. "Restriction enzyme digestion" refers to catalytic cleavage of a nucleic acid molecule with an enzyme that functions only at specific locations in a nucleic acid molecule. "Ligation" refers to the process of forming phosphodiester bonds between two nucleic acid fragments, which may or may not be contiguous with each other. Techniques involving restriction enzymes and ligation reactions are well known to those of skill in the art of recombinant technology.

### C. Origins of Replication

In order to propagate a vector in a host cell, it may contain one or more origins of replication sites (often termed "ori"), for example, a nucleic acid sequence corresponding to oriP of EBV as described above or a genetically engineered oriP with a similar or elevated function in reprogramming, which is a specific nucleic acid sequence at which replication is initiated. Alternatively a replication origin of other extra-chromosomally replicating virus as described above or an autonomously replicating sequence (ARS) can be employed.

### D. Internal Ribosome Entry Sites (IRES) and Protease Cleavage Sites/Self-Cleaving Peptides

IRES sequences are included in aspects of the embodiments to allow polycistronic transcripts to be produced. An IRES sequence allows simultaneous expression of multiple proteins from a single genetic locus. IRES elements can be linked to heterologous open reading frames. Multiple open reading frames can be transcribed together, each separated by an IRES, creating polycistronic messages. By virtue of the IRES element, each open reading frame is accessible to ribosomes for efficient translation. Multiple genes can be efficiently expressed using a single promoter/enhancer to transcribe a single message. In some cases, IRES-dependent polycistronic systems may be used to enhance the efficiency of producing iPS cells.

One embodiment involves including coding sequences for both a desired recombinant product and a reporter within the same polycistronic transcript. Successful transformation events are marked by both expression of the desired reprogramming factors and the easily detectable reporters, facilitating selection of successfully transfected cells.

### E. Reporters

In certain embodiments, cells containing a nucleic acid construct of the embodiments may be identified *in vitro* or *in vivo* by including a marker in the expression vector. Such markers would confer an identifiable change to the cell permitting easy identification of cells containing the expression vector. Generally, a selection marker is one that confers a property that allows for selection. A positive selection marker is one in which the presence of the marker allows for its selection, while a negative selection marker is one in which its presence prevents its selection. An example of a positive selection marker is a drug resistance marker.

Usually the inclusion of a drug selection marker aids in the cloning and identification of transformants, for example, genes that confer resistance to neomycin, puromycin, hygromycin, DHFR, GPT, zeocin and histidinol are useful selection markers. In addition to markers conferring a phenotype that allows for the discrimination of transformants based on the implementation of conditions, other types of markers including screenable markers, such as fluorescent proteins and β-galactosidase, are also contemplated. Alternatively, screenable enzymes as negative selection markers, such as herpes simplex virus thymidine kinase (tk) or chloramphenicol acetyltransferase (CAT), may be utilized. One of skill in the art would also know how to employ immunologic markers (*e.g*., cell surface proteins), possibly in conjunction with FACS analysis. The marker used is not believed to be important, so long as it is capable of being expressed simultaneously with the nucleic acid encoding a gene product. Further examples of selection and screenable markers are well known to one of skill in the art. One feature of the embodiments includes using selection and screenable markers to select vector-free cells after the reprogramming factors have effected the reprogramming of the cells. An additional advantage is to enrich induced pluripotent stem cells with silenced reporter expression.

### F. Vector Delivery

Introduction of a reprogramming vector into somatic cells according to the embodiments may use any suitable methods for nucleic acid delivery for transformation of a cell, as described herein or as would be known to one of ordinary skill in the art. Such methods include, but are not limited to, direct delivery of DNA such as by *ex vivo* transfection; by injection, including microinjection; by electroporation; by calcium phosphate precipitation; by DEAE-dextran followed by polyethylene glycol; by direct sonic loading; by liposome-mediated transfection; by receptor-mediated transfection; by microprojectile bombardment; by agitation with silicon carbide fibers; by Agrobacterium-mediated transformation; by PEG-mediated transformation of protoplasts; by desiccation/inhibition-mediated DNA uptake; and any combination of such methods. Through the application of techniques such as these, cells may be stably or transiently transformed.

### V. Kits of the Embodiments

Any of the compositions described herein may be comprised in a kit. In some embodiments, HLA-A^{neg} iPSCs are provided in the kit, which also may include reagents suitable for expanding and/or differentiating the cells, such as media, aAPCs, growth factors, antibodies (*e.g*., for sorting or characterizing cells) and/or plasmids encoding CARs or transposase.

In a non-limiting example, the kit comprises a chimeric receptor expression construct, one or more reagents to generate a chimeric receptor expression construct, cells for transfection of the expression construct, and/or one or more instruments to obtain allogeneic cells for transfection of the expression construct (such an instrument may be a syringe, pipette, forceps, and/or any such medically approved apparatus).

In some embodiments, an expression construct for eliminating endogenous HLA-A expression, one or more reagents to generate an expression construct, and/or a CAR expression construct are provided in the kit. In some embodiments, there includes expression constructs that encode zinc finger nuclease(s), TALEN, or CRISPR/Cas9.

In some aspects, the kit comprises reagents or apparatuses for electroporation of cells.

The kits may comprise one or more suitably aliquoted compositions of the embodiments or reagents to generate compositions of the embodiments. The components of the kits may be packaged either in aqueous media or in lyophilized form. The container means of the kits may include at least one vial, test tube, flask, bottle, syringe, or other container means, into which a component may be placed, and preferably, suitably aliquoted. Where there is more than one component in the kit, the kit also will generally contain a second, third, or other additional container into which the additional components may be separately placed. However, various combinations of components may be comprised in a vial. The kits of the embodiments also will typically include a means for containing the cells, constructs, and any other reagent containers in close confinement for commercial sale. Such containers may include injection or blow molded plastic containers into which the desired vials are retained, for example.

### VI. Examples

The following specific and non-limiting examples are to be construed as merely illustrative, and do not limit the present disclosure in any way whatsoever. Without further elaboration, it is believed that one skilled in the art can, based on the description herein, utilize the present disclosure to its fullest extent. Where reference is made to a URL or other such identifier or address, it is understood that such identifiers can change and particular information on the internet can come and go, but equivalent information can be found by searching the internet. Reference thereto evidences the availability and public dissemination of such information.

### Example 1 - Generation of HLA-A^{neg} hematopoietic stem cells

The clinical use of registered NMDP donors could be markedly increased if allogeneic HSCs were genetically edited to eliminate expression of the HLA-A locus. Engineered zinc finger nucleases (ZFNs) (or any artificial nuclease, *e.g*., TALEN, CRISPR/Cas9) can be used to eliminate HLA-A expression in genetically edited T cells and cell lines. To extend genetic editing to hematopoietic stem cells (HSCs), HLA-A expression was disrupted by introducing ZFNs targeting this locus. CD34+lineage^{neg} HSCs (99% purity) were isolated from umbilical cord blood (UCB) by first depleting lineage^{pos} cells using a mixture of biotinylated anti-human antibodies against CD2, CD3, CD11b, CD14, CD15, CD16, CD19, CD56, CD61, and CD235a (Glycophorin A) and depleting using biotin-binding paramagnetic beads. Next, CD34+ cells were isolated using anti-CD34 magnetic beads. Electro-transfer of *in vitro*-transcribed mRNA species encoding the HLA-A-specific ZFNs generated 30% HLA-A^{neg} HSCs after one week *ex vivo* culture with defined cytokines (FLT3-L, SCF, TPO, and IL-6) and an aryl hydrocarbon receptor antagonist (stem reginin-1, SR-1). DNA sequence analysis revealed that HLA-A^{neg} HSCs displayed the expected nucleotide changes at the ZFN target site. *In vitro* assays showed no significant difference in lineage-specific colony formation between HLA-A^{pos} HSC and HLA-A^{neg} HSC. Moreover, an *in vivo* engraftment assay, using NOD.Cg*-Prkdc^{scid} Il2rg*^{*tm*/*Wjl*}/*S*zJ (NSG) mice, demonstrated that engineered HLA-A^{neg} HSCs maintain the capability of engraftment and differentiation into HLA-A^{neg} multi-lineage hematopoietic cells.

### Example 2 - Generation of HLA-A^{neg} iPSC

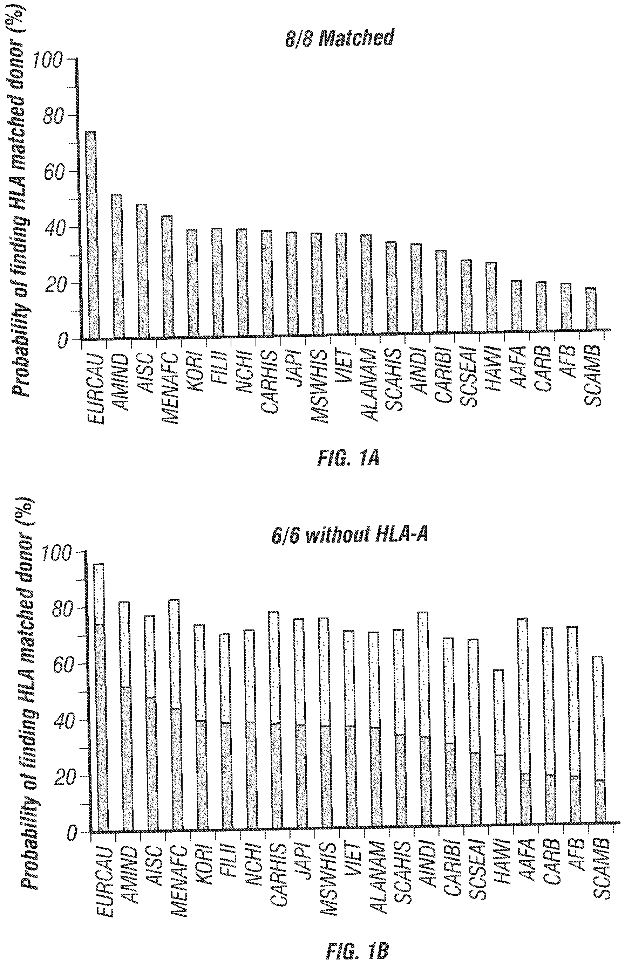

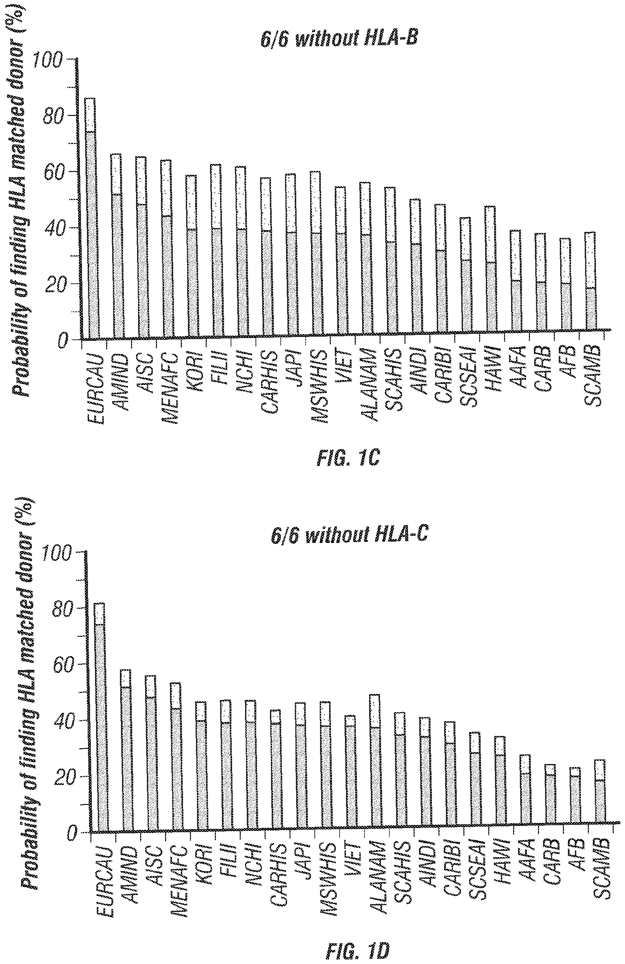

One way to minimize the number of donors needed may be the elimination of one HLA locus from an HLA-homozygous donor. It has been determined that HLA-A elimination from donor cells may have a significant impact on the chance of finding suitable HLA-matched donors (FIG. 1). Therefore, elimination of HLA-A expression from HLA homozygous donors will decrease the needed number of banked iPSCs that can be infused into HLA-matched patients. Further elimination of endogenous TCR αβ and γδ expression, which causes unwanted allogeneic immune reaction, may be performed to.reduce graft-versus-host disease. The iPSC can be further modified to express a suicide gene (*e.g.,* iCasp9, inducible caspase 9) to secure safety of allogeneic iPSC therapy for disease and regenerative medicine. iPSC clones will be isolated that have the "safe harbor" genetic profile (Papapetrou *et al.,* 2011) as assayed by whole genome sequencing and integration site analyses (*e.g*., an integration site where transgene expression is sustained and does not disrupt expression of endogenous genes). This suicide gene⁺, TCR^{neg}, HLA-A^{neg}, HLA-homozygous iPSC bank can be used to prepare allogeneic cell products useful in a wide range of patients. This iPSC bank will improve therapeutic potential, enabling a limited starting pool of cells for administration into a large number of recipients. One potential immediate application of these iPSCs will be i) immune cells (including T cells (Themeli *et al.,* 2013), NK cells (Knorr *et al.,* 2013), NKT cells, *etc.*) that express tumor- or virus-specific immune receptors (*e.g*., TCRαβ or chimeric antigen receptor (CAR)) for immunotherapy and ii) hematopoietic stem cell transplantation for hematologic malignancies or congenital disorders. In addition, allogeneic iPSC can be used to generate, for example, cardiomyocytes, lung epithelial cells, renal cells, and neuronal cells for regenerative medicine. iPSC can also be used for safe cellular engineering due to the relatively high efficiency for cloning and robust proliferation. For example, iPSC can be modified to express therapeutic genes (*e.g.,* CAR) and also genetically edited by artificial nucleases (*e.g*., ZFN, TALEN, CRISPR/Cas9) to eliminate immune suppressive molecules (*e.g.,* PD-1, CTLA-4, TCR alpha constant region).

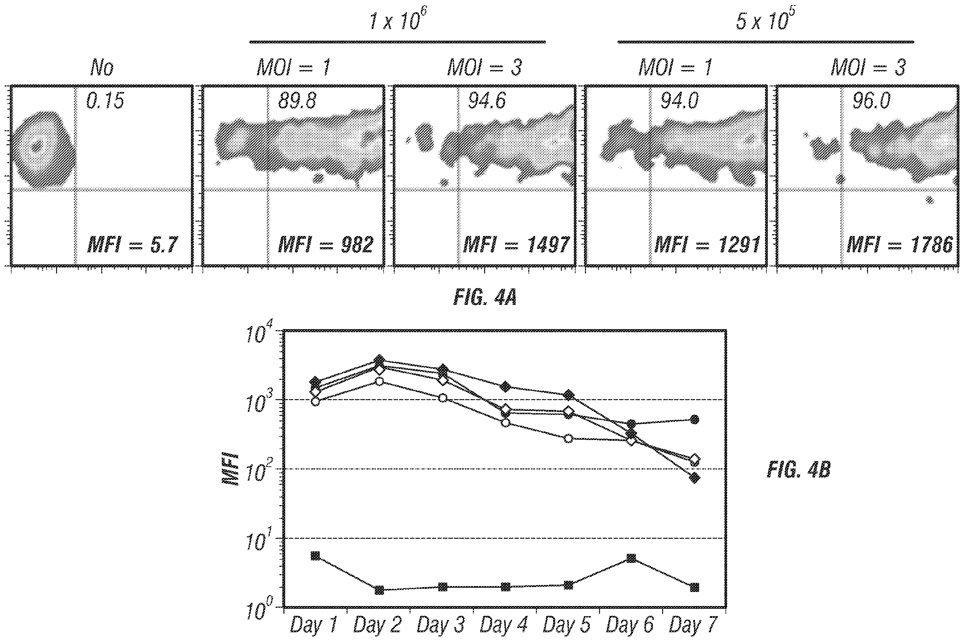

*Sendai virus vector-mediated gene transduction into T cells.* Sendai virus vector was used for generating iPSCs from T cells derived from HLA homozygous UCB (Table 2). First, the efficiency of SeV was tested in T cells by using SeV vector carrying a GFP reporter gene. Two days after activation of T cells with anti-CD3 monoclonal antibody (moAb) and anti-CD28 moAb, the cells were infected GFP-SeV particles. GFP expression from SeV was extremely high and its expression was maintained after a week (almost 3 log higher MFI than non-transfected T cells) (FIG. 4).

**Table 2. HLA Homozygous UCB for iPS**

| CB-ID | Gender | HLA-A | | HLA-B | | HLA-C | | HLA-DRB1 | | HLA/B/C/DRB1 haplo frequency | | |
|---|---|---|---|---|---|---|---|---|---|---|---|---|
| | | AL-RAW | A2-RAW | B1_RAW | B2_RAW | C1_RAW | 2_RAW | DRB1_1_RAW | DRB1_2_RAW | EUR_freq | Rank | Population freq |
| 3213 | Female | A02:xx | A02:xx | B08:xx | B08:xx | C07:xx | C07:xx | DRB1*03:01:01 | DRB1*03:01:01 | 0.10285 | 1 | 0.2057 |
| 3233 | Female | A03:xx | A03:xx | B07:xx | B07:xx | C07:xx | B07:xx | DRB1*15:01:01 | DRB1*15:01:01 | 0.08171 | 2 | 0.16342 |
| 23707 | Female | A02:EZFZ | A02:EZFZ | B:07:EYCB | B07:EYCB | C04:FDZZ | C07:FEAA | DRB1*15:01 | DRB1*15:01 | 0.08171 | 2 | 0.16342 |
| 9855 | Female | A02:xx | A03:xx | B35:xx | B35:xx | C04:xx | C04:xx | DRB1*01:01:01 | DRB1*01:01:01 | 0.02745 | 5 | 0.0549 |
| 10363 | Female | A23:xx | A33:GN | B44:ARXV | B44:xx | CO4:xx | C04:xx | DRB1*07:01 | DRB1*07:01 | 0.01144 | 12 | 0.02288 |

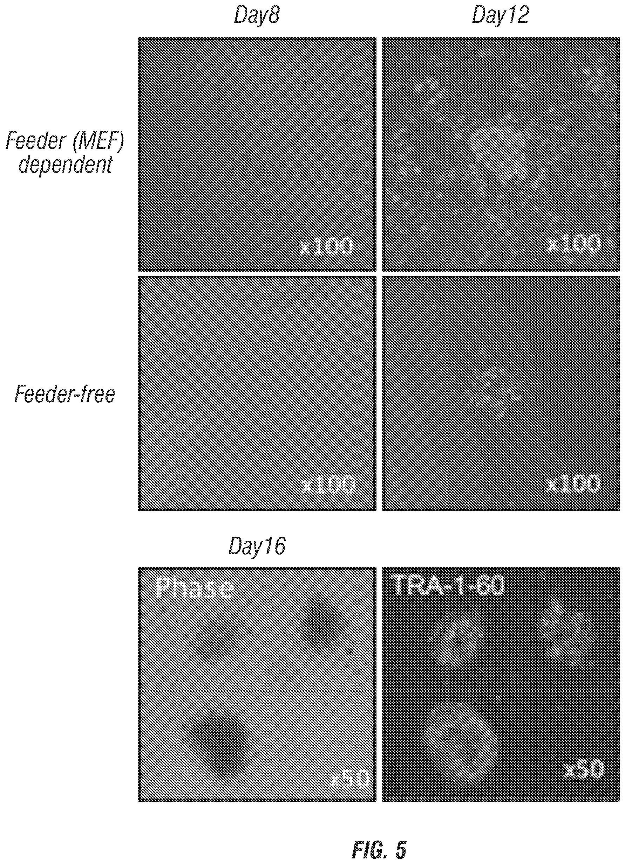

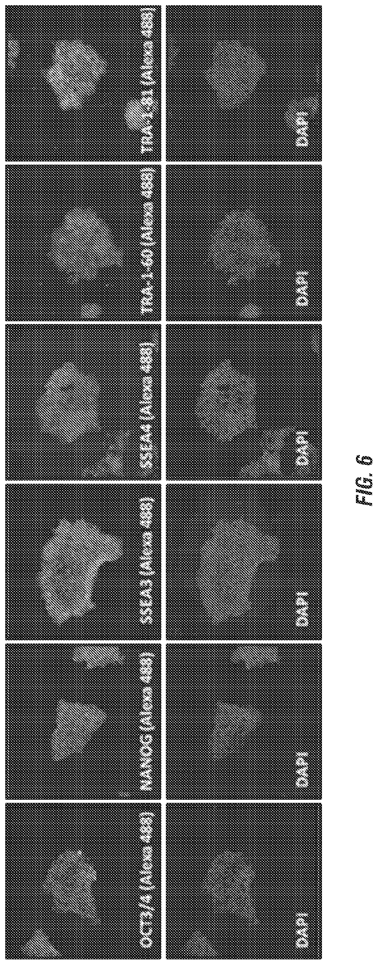

*Generation of iPSCs from T cells derived from HLA homozygous UCB.* An HLA homozygous UCB unit was obtained from the Cord Blood Bank at MD Anderson Cancer Center. In one method, after isolation of T cells, the cells were infected with SeV vectors encoding i) KLF4, OCT4, SOX2 (polycistronic vector), ii) cMYC, and iii) KLF4 at MOI (5, 5, 3, respectively). After 12 days on MEF feeder cultures or Matrigel, iPS colonies emerged and these colonies were evaluated with TRA-1-60 staining on day 16 (FIG. 5). After initial induction on MEF feeder condition, iPSCs were further cultured on feeder free (Matrigel coated) plates with mTeSR1 medium. iPSCs maintained pluripotent cell like morphology in this condition, and the expression of pluripotent markers by the iPSCs was confirmed with immunofluorescence staining (FIG. 6).

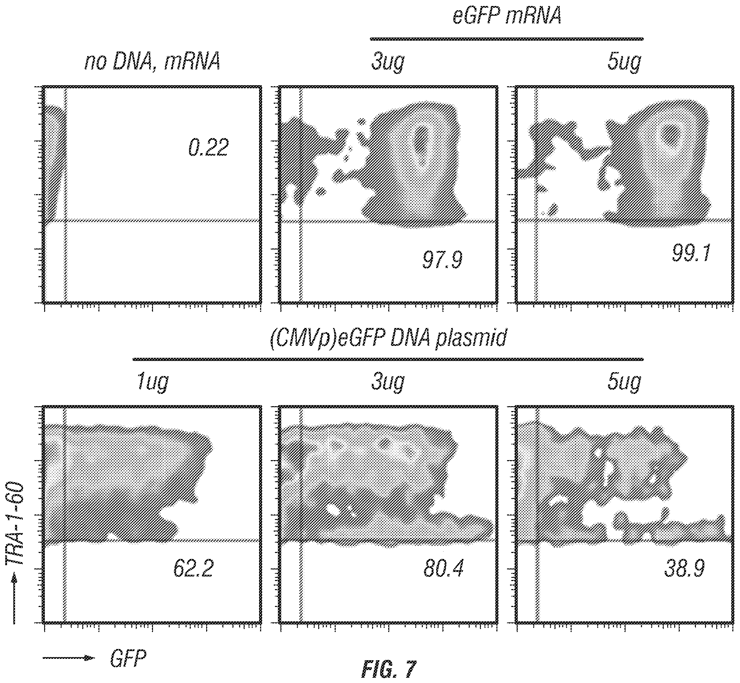

*Electroporation of DNA plasmid or in vitro transcribed mRNA into iPSC.* The Sleeping Beauty transposon/transposase system was adopted to generate chimeric antigen receptor expressing T cells for adoptive immunotherapy against cancer. This system is much less expensive than virus-based genetic modification. Because this system relies on the transfection of DNA plasmid (or mRNA) by Nucleofection, Nucleofection was tested in iPSCs with a DNA plasmid encoding CMV promoter-driven GFP and an mRNA encoding GFP. With 4D Amaxa Nucleofector (Program: CA-137), DNA plasmid and mRNA were introduced into iPSCs (FIG. 7). This high transfection efficiency was achieved when using small scale transfection (20 µL) using strips.

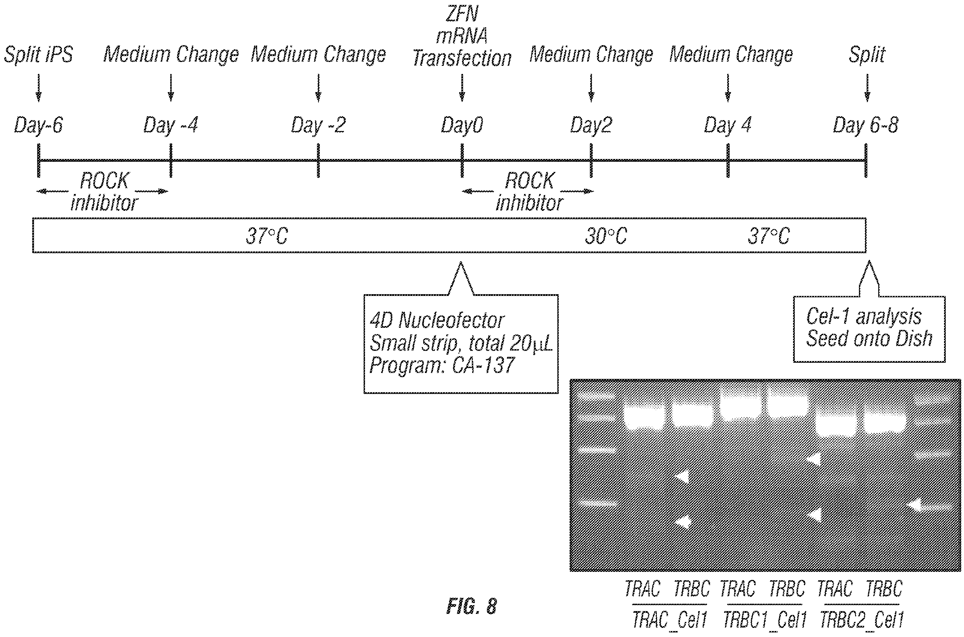

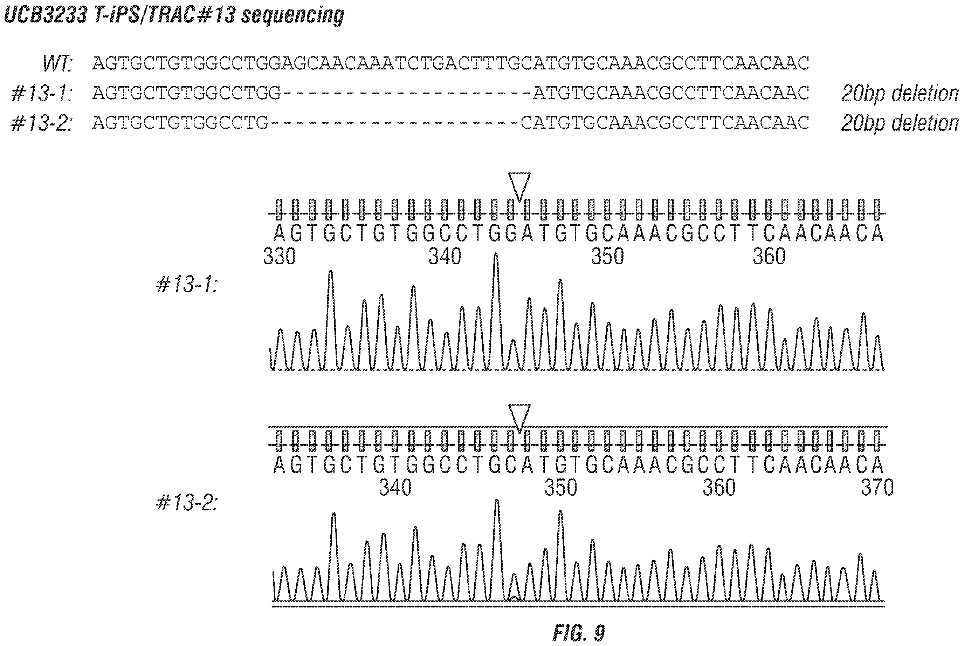

*Disrupting TRAC gene by zinc finger nuclease.* FIG. 8 provides an exemplary protocol for disrupting the TRAC gene (TCR alpha constant region) by transfection of an mRNA-encoded zinc finger nuclease. TRAC gene disrupted iPS clones were isolated and analyzed by genomic DNA sequencing to identify clones with disrupted a TRAC gene (FIG. 9). TRAC gene disrupted iPS clones were found to express pluripotent markers including OCT3/4, Nanog, SSEA3, SSEA4, TRA-1-60, and TRA-1-81.

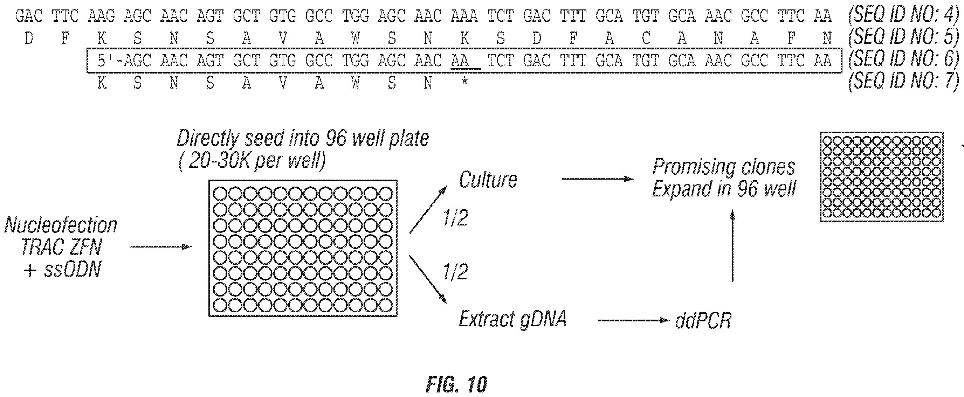

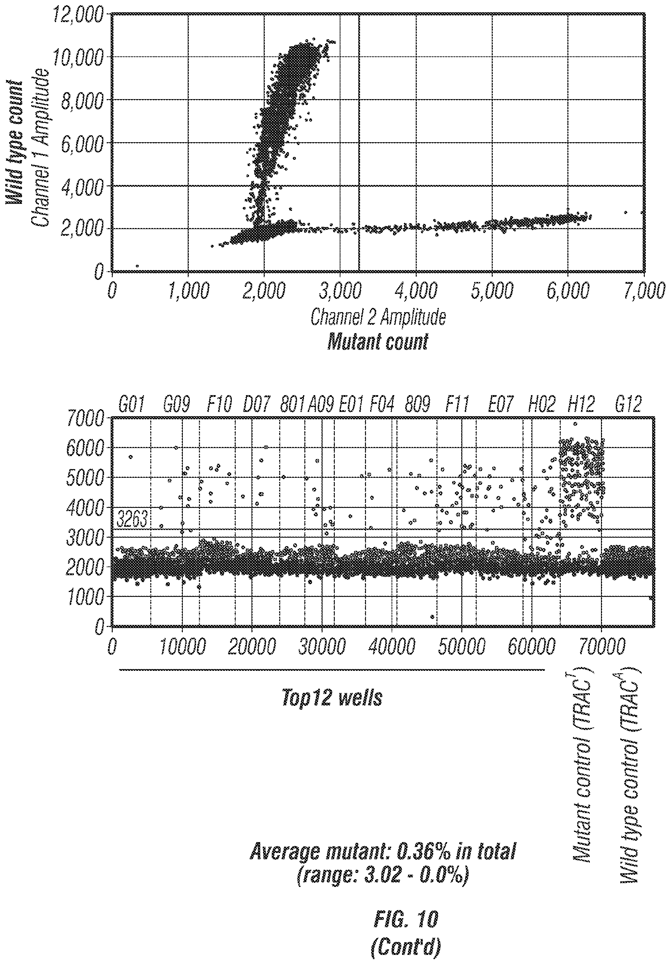

*Disrupting the TCR gene by zinc finger nuclease.* Zinc finger nucleases were used in conjunction with single-stranded donor oligonucleotides (ssODN) (Chen et al., 2011) to disrupt the TCR gene is iPS cells. Then, mutants were screened using droplet digital PCR (ddPCR) (Miyakoa et al., 2014) (FIG. 10).

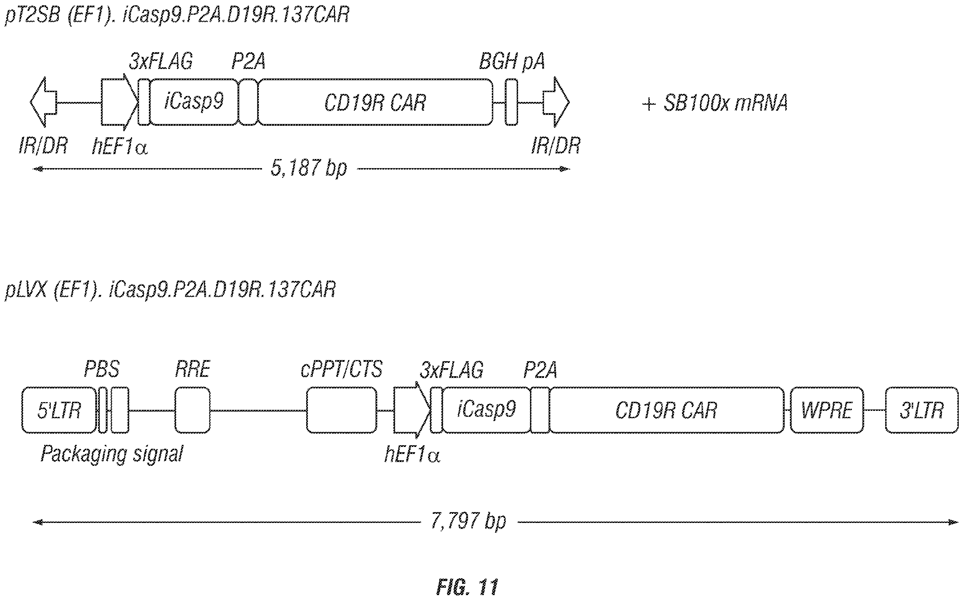

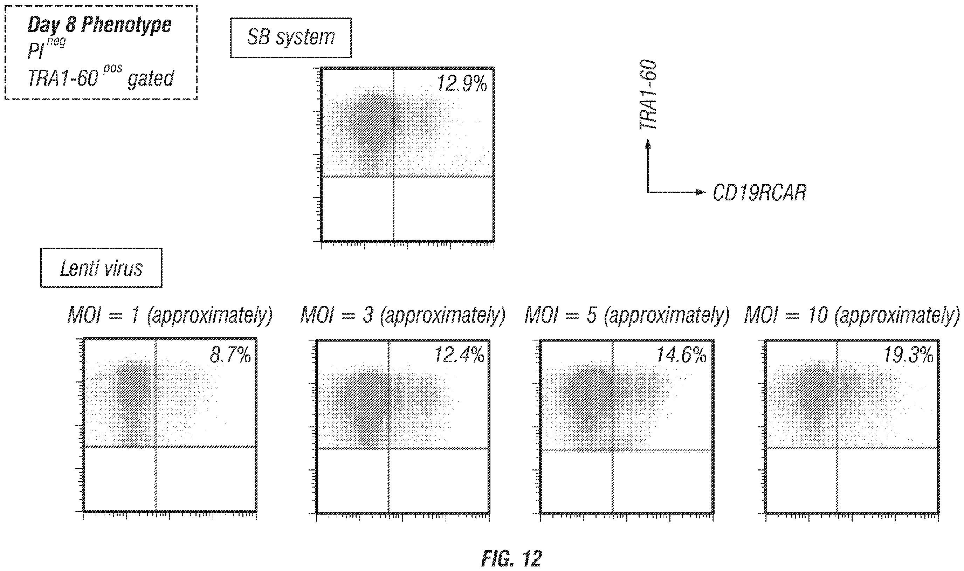

*Introduction of CAR.* Introduction of CAR was performed using Sleeping Beauty and Lentivirus (FIG. 11). SB-mediated CAR transduction was found to be comparable to lentivirus-mediated CAR transduction in iPS cells (FIG. 12). Positive selection with anti-FC (detecting CAR with IgG stalk) induced differentiation or death of iPS cells may be used to enrich the CAR⁺ population.

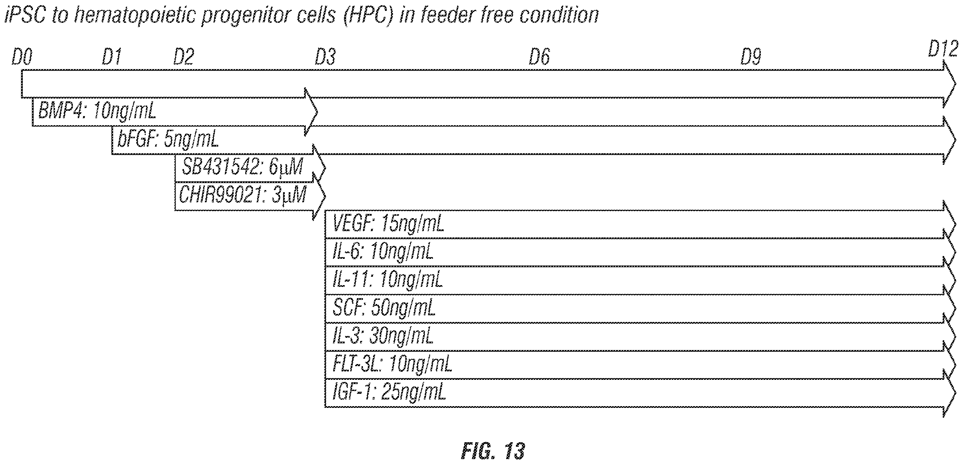

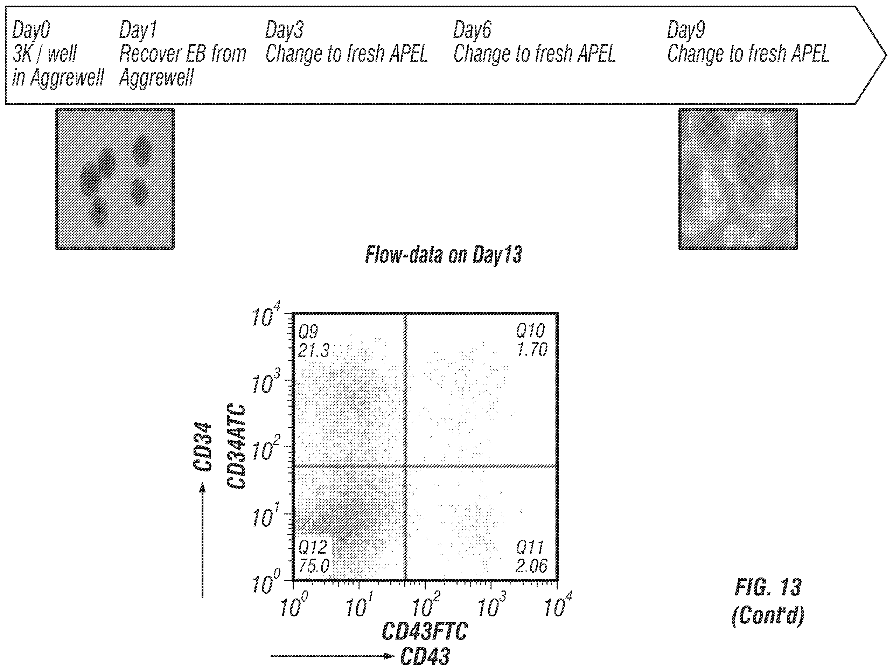

*Re-differentiation of T cells from iPS cells.* FIG. 13 provides an exemplary protocol for differentiation of hematopoietic progenitor cells (HPC) from iPS cells in feeder free conditions.

### Example 3 - Reporter gene mediated screening of potential off-target toxicity of immune receptors

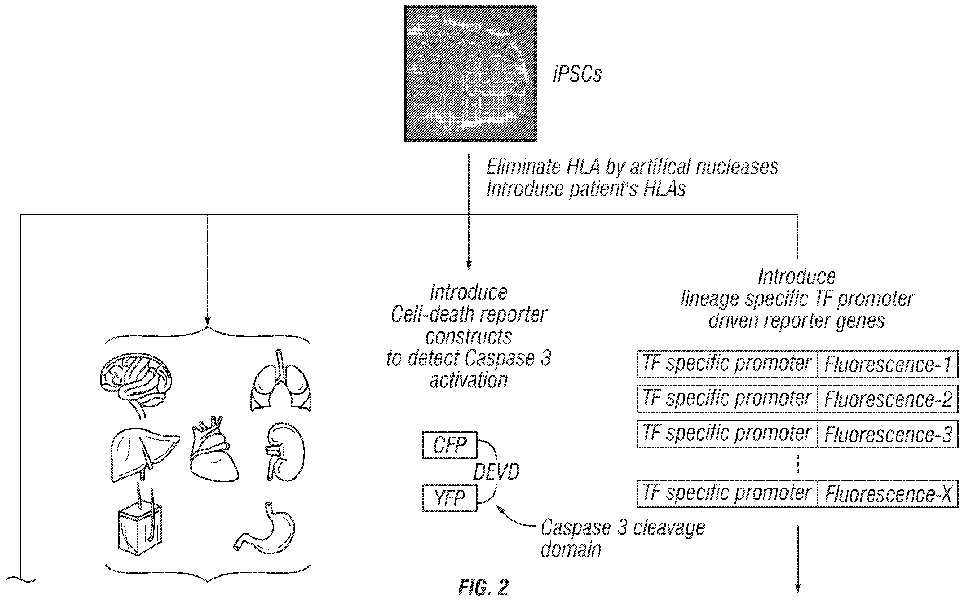

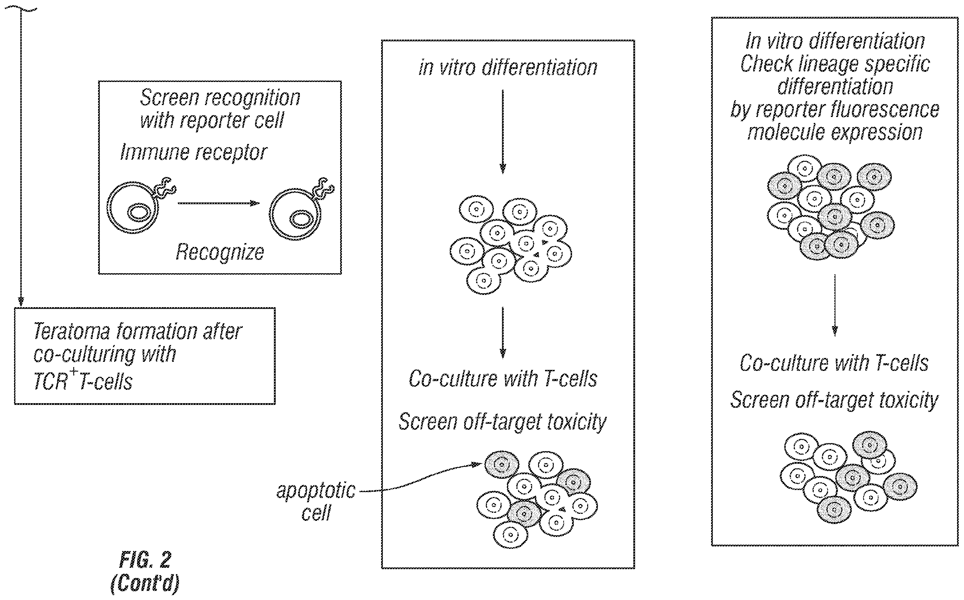

Adoptive T-cell therapy using immune receptors targeting tumors has been emerging in the clinic due to its potential to eradicate tumor cells. However, a major concern for redirecting immune cell specificity to a tumor is the unintended and unpredictable off-target toxicities, which have been observed in patients who received "tumor-specific" adoptive cell therapy (Cameron *et al.,* 2013; Morgan *et al.,* 2010). The current methods to predict tissue distribution of target antigens based on the detection of gene or protein expression in tissue panels are not enough to predict these toxicities. To overcome this issue and improve the identification of potential off-target toxicities, the inventors will screen specificities of immune receptors using iPSCs and/or iPSC-derived differentiated cells. First, iPSCs will be generated from healthy donor cells and endogenous HLA expression will be eliminated by artificial nucleases (*e.g*., zinc finger nuclease, TALEN, or CRISPR/Cas9) to reduce the background recognition of immune receptors of interest. These iPSC will be differentiated to lineage-specific cells and screened using reporter cell lines derived from T-acute lymphoblastic leukemia cell lines that have endogenous TCR expression eliminated and an introduced immune receptor of interest (FIG. 2, left panel). This reporter cell line will express GFP only after recognizing a target antigen through the introduced immune receptor. Therefore, if iPSC-derived differentiated cells express target antigens, they can be detected by GFP expression. Alternatively, death reporter genes (FIG. 2, middle panel) or lineage-specific transcription factor promoter-driven reporter genes (FIG. 2, right panel) may be introduced into iPSCs. These cells can be used to screen immune receptor-mediated recognition by co-culturing with immune receptor-expressing T cells.

### REFERENCES

U.S. Patent No. 4,690,915
U.S. Patent No. 6,225,042
U.S. Patent No. 6,355,479
U.S. Patent No. 6,362,001
U.S. Patent No. 6,458,589
U.S. Patent No. 6,506,574
U.S. Patent No. 6,790,662
U.S. Patent No. 6,833,269
U.S. Patent No. 7,109,304
U.S. Patent No. 7,250,294
U.S. Patent No. 7,285,415
U.S. Patent No. 7,473,555
U.S. Patent No. 7,449,334
U.S. Patent No. 7,763,464
U.S. Patent No. 7,897,389
U.S. Patent No. 7,955,849
U.S. Patent No. 7,993,638
U.S. Patent No. 8,124,408
U.S. Patent No. 8,268,620
U.S. Patent No. 8,283,168
U.S. Patent No. 8,372,642
U.S. Patent No. 8,415,155
U.S. Patent No. 8,546,140
U.S. Patent No. 8,557,580
U.S. Patent No. 8,951,798
U.S. Patent Application Publication No. 2003/0153082
U.S. Patent Application Publication No. 2009/0004142
U.S. Patent Application Publication No. 2012/0276063
WO 03/004605
WO 03/050249
WO 12/109208
Ausubel et al., Current Protocols in Molecular Biology, Greene Publ. Assoc. Inc. & John Wiley & Sons, Inc., MA, 1994.
Boyer et al., Core Transcriptional Regulatory Circuitry in Human Embryonic Stem Cells, Cell, 122: 947-956, 2005.
Brambrink, et al, Cell Stem Cell, 2:151-159, 2008.
Cameron et al., Identification of a Titin-derived HLA-A1-presented peptide as a cross-reactive target for engineered MAGE A3-directed T cells, Sci. Transl. Med., 5(197):197ra103, 2013.
Carpenter et al., Enrichment of neurons and neural precursors from human embryonic stem cells, Exp. Neurol., 172:383-397, 2001.
Chadwick et al., Blood, 102:906-915, 2003.
Chen et al., Nat. Methods, 8:424-429, 2011.
Chen et al., Nat. Methods, 8:753-755, 2011.
Jaenisch, Science, 240:1468-1474, 1988.
Keirstead et al., Human embryonic stem cell-derived oligodendrocyte progenitor cell transplants remyelinate and restore locomotion after spinal cord injury, J. Neurosci., 25:4694-4705 2005.
Kim et al., Nature, 22:403-410, 2004.
Klimanskaya et al., Derivation and comparative assessment of retinal pigment epithelium from human embryonic stem cells using transcriptomics, Cloning and Stem Cells, 6:217-245, 2004.
Knorr et al., Clinical-scale derivation of natural killer cells from human pluripotent stem cells for cancer therapy, Stem Cells Transl. Med., 2:274-283, 2013.
Li et al., Genetic correction using engineered nucleases for gene therapy applications, Development, Growth & Differentiation, 56(1):63-77, 2013.
Ludwig et al., Nat. Methods, 3:637-646, 2006a.
Ludwig et al, Nat. Biotechnol., 24:185-187, 2006b.
Miyaoka et al., Nat. Methods, 11:291-293, 2014.
Morgan et al., Case report of a serious adverse event following the administration of T cells transduced with a chimeric antigen receptor recognizing ERBB2, Mol. Ther., 18:843-851,2010.
Narazaki et al., Circulation, 118:498-506, 2008.
Ng et al., Development, 132:873-884, 2005.
Okita et al., A more efficient method to generate integration-free human iPS cells, Nat. Methods, 8:409-412, 2011.
Papapetrou et al., Genomic safe harbors permit high beta-globin transgene expression in thalassemia induced pluripotent stem cells, Nat. Biotechnol., 29:73-78, 2011.
Riolobos et al., HLA Engineering of Human Pluripotent Stem Cells, Amer. Soc. Gene & Cell Therapy, 21(6):1232-1241, 2013.
Sambrook and Russell, Molecular Cloning: A Laboratory Manual 3rd Ed., Cold Spring Harbor Laboratory Press, 2001.
Schneider, J. Embryol. Exp. Morph., 27:353-365, 1972.
Stadtfeld et al., Cell Stem Cell, 2:230-240, 2008.
Themeli et al., Generation of tumor-targeted human T lymphocytes from induced pluripotent stem cells for cancer therapy, Nat. Biotechnol., 31:928-933, 2013.
Topalian and Rosenberg, Acta Haematol., 78(Suppl 1):75-76, 1987.
Torikai et al., A foundation for universal T-cell based immunotherapy: T cells engineered to express a CD19-specific chimeric-antigen-receptor and eliminate expression of endogenous TCR, Blood, 119:5697-5705, 2012.
Torikai et al., Towards eliminating HLA class I expression to generate universal cells from allogeneic donors, Blood, 122:1341-1349, 2013.
Wang et al., Nat. Biotechnol., 25:317-318, 2007.
Wang et al., Generation of induced pluripotent stem cells with high efficiency from human umbilical cord blood mononuclear cells, Genomics Proteomics Bioinformatics, 11:304-311, 2013.
Yamanaka et al., Cell, 131:861-872, 2007.
Yu et al., Science, 318:1917-1920, 2007.
Zhang et al., Circ. Res., 104:e30-41, 2009.
Zhou et al, Cell Stem Cell, 4:381-384 2009.

## Claims

1. A method of producing HLA-A^{neg}, HLA-homozygous induced pluripotent stem cells (iPSCs) comprising:
(a) obtaining a population of umbilical cord blood cells from an HLA-homozygous donor, wherein the donor is HLA-homozygous at HLA-B, HLA-C, and HLA-DRB 1;
(b) engineering the cells so that they do not express HLA-A, thereby producing HLA-A^{neg} cells; and
(c) reprogramming the HLA-A^{neg} cells to generate iPSCs, thereby producing HLA-A^{neg} iPSCs.

2. The method of claim 1, wherein reprogramming comprises introducing Oct3/4, KLF4, Sox2, and c-myc encoding mRNA into the cells.

3. The method of claim 1, wherein reprogramming comprises introducing one or more expression cassettes encoding Oct3/4, KLF4, Sox2, and c-myc into the cells.

4. The method of claim 1, further comprising transducing the iPSCs with a chimeric antigen receptor.

5. The method of claim 1, further comprising differentiating the HLA-A^{neg}, HLA-homozygous iPSCs.

6. The method of claim 5, wherein the iPSCs are differentiated into immune cells.

7. The method of claim 5, wherein the iPSCs are differentiated into hematopoietic stem cells.

8. A mammalian cell, said cell comprising at least one set of homozygous HLA alleles and a HLA-A^{neg} phenotype.

9. The cell of claim 8, wherein the cell is an umbilical cord blood, cardiomyocyte, kidney, lung, epidermal, pancreatic, beta islet, liver, hematopoietic, mesenchymal, neural cell, T cell, NK cell, embryonic stem cell or iPS cell.

10. The cell of any one of claims 8 or 9, wherein cell comprises homozygous HLA-B and HLA-C alleles, homozygous HLA-B and HLA-DRB1 alleles or homozygous HLA-C and HLA-DRB1 alleles.

11. The cell of any one of claims 8-10, wherein the cell further comprise a chimeric antigen receptor (CAR).

12. A population of cells in accordance with any one of claims 8-11.

13. An *in vitro* set of cell lines comprising at least a first and second induced pluripotent stem cell line, wherein said first and second lines comprise homozygous HLA-B and HLA-C alleles, wherein the homozygous HLA-B and/or HLA-C alleles of the first cell line are different from the homozygous HLA-B and/or HLA-C alleles of the second cell line, wherein the first and second line are both HLA-A^{neg}.

14. A method of screening immune receptor specificity comprising:
(a) obtaining an *in vitro* set of iPSC lines according to claim 13;
(b) optionally differentiating the iPSCs into lineage-specific cells;
(c) exposing the iPSCs or lineage-specific cells to T cells expressing an immune receptor of interest; and
(d) detecting an interaction between the iPSCs or lineage-specific cells and the T cells expressing an immune receptor of interest, thereby screening for immune receptor specificity.

15. Differentiated cells for use as a medicament, wherein the use comprises;
(a) selecting a cell line from an *in vitro* set of cell lines according to claim 13 that is HLA-matched to the patient;
(b) differentiating the selected cell line to lineage-specific cells; and
(c) administering a therapeutically effective amount of the differentiated cells to the patient.

## Patentansprüche

1. Verfahren zum Herstellen von HLA-A^{neg}, HLA-homozygoten, induzierten, pluripotenten Stammzellen (iPSC), umfassend:
a) Gewinnen einer Population von Nabelschnurblutzellen von einem HLA-homozygoten Spender, wobei der Spender für HLA-B, HLA-C und HLA-DRB 1 HLA-homozygot ist,
b) Manipulieren der Zellen in einer Art, dass sie HLA-A nicht exprimieren, dadurch Herstellen von HLA-A^{neg}-Zellen und
c) Umprogrammieren der HLA-A^{neg}-Zellen, um iPSC zu erzeugen, dadurch Herstellen von HLA-A^{neg}-iPSC.

2. Verfahren nach Anspruch 1, wobei das Umprogrammieren es umfasst, mRNA, die für Oct3/4, KLF4, Sox2 und c-myx codiert, in die Zellen einzubringen.

3. Verfahren nach Anspruch 1, wobei das Umprogrammieren es umfasst, eine oder mehrere Expressionskassetten, die für Oct3/4, KLF4, Sox2 und c-myx codieren, in die Zellen einzubringen.

4. Verfahren nach Anspruch 1, weiter das Transduzieren der iPSC mit einem chimären Antigenrezeptor umfassend.

5. Verfahren nach Anspruch 1, weiter das Differenzieren der HLA-A^{neg}, HLA-homozygoten iPSC umfassend.

6. Verfahren nach Anspruch 5, wobei die iPSC zu Immunzellen differenziert werden.

7. Verfahren nach Anspruch 5, wobei die iPSC zu hämatopoetischen Stammzellen differenziert werden.

8. Säugerzelle, wobei besagte Zelle mindestens einen Satz homozygoter HLA-Allele und einen HLA-A^{neg} Phänotyp umfasst.

9. Zelle nach Anspruch 8, wobei die Zelle eine Nabelschnurblut-, Kardiomyozyten-, Nieren-, Lungen-, epidermale, Pankreas-, Beta-Insel-, Leber-, hämatopoetische, mesenchymale, neurale Zelle, T-Zelle, NK-Zelle, embryonale Stammzelle oder iPS-Zelle ist.

10. Zelle nach einem der Ansprüche 8 oder 9, wobei die Zelle homozygote HLA-B und HLA-C-Allele, homozygote HLA-B und HLA-DRB1-Allele oder homozygote HLA-C und HLA-DRB1-Allele umfasst.

11. Zelle nach einem der Ansprüche 8-10, wobei die Zelle weiter einen chimären Antigenrezeptor (CAR) umfasst.

12. Zellpopulation in Übereinstimmung mit einem der Ansprüche 8-11.

13. *In-vitro*-Set von Zelllinien, mindestens eine erste und zweite induzierte, pluripotente Stammzelllinie umfassend, wobei die besagten ersten und zweiten Linien homozygote HLA-B und HLA-C-Allele umfassen, wobei die homozygoten HLA-B und/oder HLA-C-Allele der ersten Zelllinie sich von den homozygoten HLA-B und/oder HLA-C-Allelen der zweiten Zelllinie unterscheiden, wobei die erste und zweite Linie beide HLA-A^{neg} sind.

14. Verfahren zum Überprüfen der Immunrezeptorspezifität, umfassend:
a) Gewinnen eines *In-vitro*-Sets von iPSC-Linien gemäß Anspruch 13,
b) wahlweises Differenzieren der iPSC in abstammungsspezifische Zellen,
c) In-Kontakt-Bringen der iPSC oder abstammungsspezifischen Zellen mit T-Zellen, die einen Immunrezeptor von Interesse exprimieren und
d) Nachweisen einer Interaktion zwischen den iPSC oder abstammungsspezifischen Zellen mit den T-Zellen, die einen Immunrezeptor von Interesse exprimieren, dadurch Überprüfen auf Immunrezeptorspezifität.

15. Differenzierte Zellen zur Verwendung als Medikament, wobei die Verwendung umfasst:
a) Auswählen einer Zelllinie, die dem Patienten in Bezug auf HLA angepasst ist, aus einem *In-vitro*-Set von Zelllinien gemäß Anspruch 13,
b) Differenzieren der ausgewählten Zelllinie in abstammungsspezifische Zellen und
c) Verabreichen einer therapeutisch wirksamen Menge der differenzierten Zellen an den Patienten.

## Revendications

1. Procédé de production de cellules souches pluripotentes induites (iPSC) homozygotes-HLA, HLA-A^{neg} comprenant :
(a) l'obtention d'une population de cellules du sang du cordon ombilical d'un donneur homozygote-HLA, le donneur étant homozygote-HLA au niveau de HLA-B, HLA-C, et HLA-DRB 1 ;
(b) la construction génétique des cellules de sorte qu'elles n'expriment pas HLA-A, produisant ainsi des cellules HLA-A^{neg} ; et
(c) la reprogrammation des cellules HLA-A^{neg} pour générer des iPSC, produisant ainsi des HLA-A^{neg} iPSC.

2. Procédé selon la revendication 1, la reprogrammation comprenant l'introduction d'ARNm codant pour Oct3/4, KLF4, Sox2, et c-myc dans les cellules.

3. Procédé selon la revendication 1, la reprogrammation comprenant l'introduction d'une ou plusieurs cassettes d'expression codant pour Oct3/4, KLF4, Sox2, et c-myc dans les cellules.

4. Procédé selon la revendication 1, comprenant en outre la transduction des iPSC avec un récepteur d'antigène chimère.

5. Procédé selon la revendication 1, comprenant en outre la différenciation des iPSC homozygotes-HLA, HLA-A^{neg}.

6. Procédé selon la revendication 5, les iPSC étant différenciées en cellules immunes.

7. Procédé selon la revendication 5, les iPSC étant différenciées en cellules souches hématopoïétiques.

8. Cellule de mammifère, ladite cellule comprenant au moins un ensemble d'allèles HLA homozygotes et un phénotype HLA-A^{neg}.

9. Cellule selon la revendication 8, la cellule étant une cellule du sang du cordon ombilical, un cardiomyocyte, une cellule rénale, une cellule pulmonaire, une cellule de l'épiderme, une cellule pancréatique, une cellule des îlots bêta, une cellule hépatique, une cellule hématopoïétique, une cellule mésenchymateuse, une cellule neurale, une cellule T, une cellule NK, une cellule souche embryonnaire ou une cellule iPS.

10. Cellule selon l'une quelconque des revendications 8 ou 9, la cellule comprenant des allèles HLA-B et HLA-C homozygotes, des allèles HLA-B et HLA-DRB1 homozygotes ou des allèles HLA-C et HLA-DRB1 homozygotes.

11. Cellule selon l'une quelconque des revendications 8 à 10, la cellule comprenant en outre un récepteur d'antigène chimère (CAR).

12. Population de cellules selon l'une quelconque des revendications 8 à 11.

13. Ensemble *in vitro* de lignées cellulaires comprenant au moins une première et une seconde lignée de cellules souches pluripotentes induites, ladite première et seconde lignée comprenant des allèles HLA-B et HLA-C homozygotes, les allèles HLA-B et/ou HLA-C homozygotes de la première lignée cellulaire étant différents des allèles HLA-B et/ou HLA-C homozygotes de la seconde lignée cellulaire, la première et la seconde lignée étant toutes deux HLA-A^{neg}.

14. Procédé de criblage de la spécificité d'un récepteur du système immunitaire comprenant :
(a) l'obtention d'un ensemble *in vitro* de lignées iPSC selon la revendication 13 ;
(b) éventuellement la différenciation des iPSC en cellules spécifiques de lignée ;
(c) l'exposition des iPSC ou des cellules spécifiques de lignée à des cellules T exprimant un récepteur d'intérêt du système immunitaire ; et
(d) la détection d'une interaction entre les iPSC ou les cellules spécifiques de lignée et les cellules T exprimant un récepteur d'intérêt du système immunitaire, effectuant ainsi le criblage de la spécificité de récepteur du système immunitaire.

15. Cellules différenciées pour l'utilisation comme médicament, l'utilisation comprenant :
(a) la sélection d'une lignée cellulaire à partir d'un ensemble *in vitro* de lignées cellulaires selon la revendication 13 à correspondance HLA pour le patient ;
(b) la différenciation de la lignée cellulaire sélectionnée en cellules spécifiques de lignée ; et
(c) l'administration d'une quantité thérapeutiquement efficace des cellules différenciées au patient.


### SEQUENCE LISTING

<110> Board of Regents, the University of Texas System
<120> APPLICATION OF INDUCED PLURIPOTENT STEM CELLS TO GENERATE OFF-THE-SHELF ADOPTIVE CELL THERAPY PRODUCTS
<130> UTFC.P1235WO
<150> US 61/983,722
   <151> 2014-04-24
<160> 7
<170> PatentIn version 3.5
<210> 1
   <211> 57
   <212> DNA
   <213> Homo sapiens
<400> 1
   agtgctgtgg cctggagcaa caaatctgac tttgcatgtg caaacgcctt caacaac 57
<210> 2
   <211> 37
   <212> DNA
   <213> Artificial sequence
<220>
   <223> Synthetic oligonucleotide
<400> 2
   agtgctgtgg cctggatgtg caaacgcctt caacaac 37
<210> 3
   <211> 37
   <212> DNA
   <213> Artificial sequence
<220>
   <223> Synthetic oligonucleotide
<400> 3
   agtgctgtgg cctgcatgtg caaacgcctt caacaac 37
<210> 4
   <211> 72
   <212> DNA
   <213> Artificial sequence
<220>
   <223> Synthetic oligonucleotide
<400> 4
   gacttcaaga gcaacagtgc tgtggcctgg agcaacaaat ctgactttgc atgtgcaaac 60
   gccttcaaca ac 72
<210> 5
   <211> 24
   <212> PRT
   <213> Artificial sequence
<220>
   <223> Synthetic peptide
<400> 5
   Asp Phe Lys Ser Asn Ser Ala Val Ala Trp Ser Asn Lys Ser Asp Phe
   1 5 10 15
   Ala Cys Ala Asn Ala Phe Asn Asn
   20
<210> 6
   <211> 60
   <212> DNA
   <213> Artificial sequence
<220>
   <223> Synthetic oligonucleotide
<400> 6
   agcaacagtg ctgtggcctg gagcaacaaa tctgactttg catgtgcaaa cgccttcaac 60
<210> 7
   <211> 10
   <212> PRT
   <213> Artificial sequence
<220>
   <223> Synthetic peptide
<400> 7
   Lys Ser Asn Ser Ala Val Ala Trp Ser Asn
   1               5                   10